(19) Europäisches Patentamt
European Patent Office
Office européen des brevets

(11) Numéro de publication: **0 064 445**
**B1**

# (12) FASCICULE DE BREVET EUROPEEN

(45) Date de publication du fascicule du brevet: **24.10.84**

(51) Int. Cl.³: **C 07 C 143/78,** C 07 D 295/12, A 61 K 31/18

(21) Numéro de dépôt: **82400717.3**

(22) Date de dépôt: **21.04.82**

## (54) Dialcoxy (2,4) benzènesulfonamides N-substitués.

(30) Priorité: **23.04.81 FR 8108125**

(43) Date de publication de la demande: **10.11.82 Bulletin 82/45**

(45) Mention de la délivrance du brevet: **24.10.84 Bulletin 84/43**

(84) Etats contractants désignés: **AT BE CH DE GB IT LI LU NL SE**

(56) Documents cités:
**FR - A - 2 141 751**
**FR - A - 2 313 918**
**GB - A - 667 772**

(73) Titulaire: **CHOAY S.A., 48, Avenue Théophile-Gautier, F-75782 Paris Cédex 16 (FR)**

(72) Inventeur: **Fournier, Jean-Paul, 10, rue Fontenay, F-78000 Versailles (FR)**
Inventeur: **Choay, Patrick, 7, Cours Jasmin, F-75016 Paris (FR)**

(74) Mandataire: **Gutmann, Ernest et al, Cabinet Plasseraud 84, rue d'Amsterdam, F-75009 Paris (FR)**

**EP 0 064 445 B1**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Description

L'invention est relative à de nouveaux composés du type benzènesulfonamides N-substitués ainsi qu'à leurs sels correspondants. L'invention est également relative à un procédé de préparation de ces composés. L'invention est enfin relative à de nouveaux médicaments contenant, à titre de principe actif, ces nouveaux composés du type benzènesulfonamides N-substitués ainsi que leurs sels obtenus avec des acides organiques ou minéraux physiologiquement acceptables. Les nouveaux benzènesulfonamides N-substitués conformes à l'invention répondent à la formule générale I:

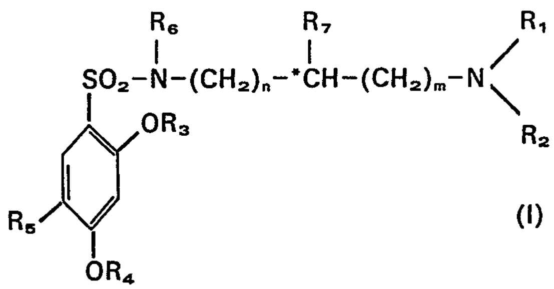

(I)

dans laquelle:

n et m, indépendamment l'un de l'autre, prennent les valeurs de 0 à 4;

$R_3$ et $R_4$ représentent chacun, indépendamment l'un de l'autre, un radical alcoyle inférieur présentant de 1 à 4 atomes de carbone;

$R_1$ et $R_2$ représentent, indépendamment l'un de l'autre, des atomes d'hydrogène, des groupes alcoyle linéaires ou ramifiés ayant de 1 à 4 atomes de carbone, ou formant conjointement avec l'atome d'azote un hétérocycle azoté à 5 ou 6 chaînons, notamment un groupe pyrrolidino, morpholino, pipérazinyle, pyrrole ou pipéridino, substitués ou non par des radicaux alcoyle linéaires ou ramifiés ayant de 1 à 4 atomes de carbone;

$R_5$ représente un atome d'hydrogène, un halogène, le groupe $NO_2$, $NH_2$ ou $CF_3$, un radical alcoxy ayant de 1 à 4 atomes de carbone, ou un radical alcoylsulfonyle ayant de 1 à 4 atomes de carbone, et

$R_6$ et $R_7$ représentent, indépendamment l'un de l'autre, un atome d'hydrogène, un radical alcoyle ayant de 1 à 6 atomes de carbone ou un radical cycloalcoyle ayant de 3 à 6 atomes de carbone.

L'invention est également relative aux sels d'addition des composés de formule I obtenus avec des acides organiques ou minéraux physiologiquement acceptables.

L'invention vise également, lorsque $R_7$ est différent de l'atome d'hydrogène, l'ensemble des isomères optiques des composés de formule I ainsi que leurs sels obtenus avec des acides organiques ou minéraux physiologiquement acceptables.

A titre de sels organiques ou minéraux physiologiquement acceptables, on peut citer par exemple le chlorhydrate, le bromhydrate, les sulfates, les phosphates, le méthanesulfonate, l'acétate, le fumarate, le succinate, le lactate, le pyruvate, le citrate, le tartrate, le maléate, le malonate, le benzoate, le salicylate, le dichloro-2,6-benzoate, le triméthoxybenzoate, le diaminobenzènesulfonate, le chromoglycate, le benzènesulfonate, le dipropylacétate et le glucose-1-phosphate.

Dans la suite du texte, les composés de formule I conformes à l'invention dans lesquels $R_5$ représente un atome d'hydrogène sont désignés par composés disubstitués, et ceux dans lesquels $R_5$ a la signification ci-dessus indiquée, à l'exception de l'hydrogène, sont désignés par des composés trisubstitués.

Une classe préférée de composés selon l'invention est constituée par les dialcoxy-(2,4)-benzènesulfonamides éventuellement substitués en positon 5 et répondant à la formule II:

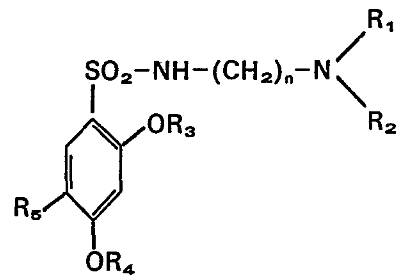

(II)

dans laquelle:

n prend les valeurs de 0 à 4, notamment 2 ou 3;

$R_3$ et $R_4$ représentent chacun, indépendamment l'un de l'autre, un radical alcoyle inférieur présentant de 1 à 4 atomes de carbone;

$R_1$ et $R_2$ représentent, indépendamment l'un de l'autre, des atomes d'hydrogène, des groupes alcoyle linéaires ou ramifiés ayant de 1 à 4 atomes de carbone, ou formant conjointement avec l'atome d'azote un hétérocycle azoté à 5 ou 6 chaînons, notamment un groupe pyrrolidino, morpholino, pipérazinyle, pyrrole ou pipéridino, substitués ou non par des radicaux linéaires ou ramifiés ayant de 1 à 4 atomes de carbone, et

$R_5$ représente un atome d'hydrogène, un halogène, le groupe $NO_2$, $NH_2$ ou $CF_3$, un radical alcoxy ayant de 1 à 4 atomes de carbone, ou un radical alcoylsulfonyle ayant de 1 à 4 atomes de carbone.

Une classe préférée de composés selon l'invention est constituée par les diméthoxy-(2,4)-benzènesulfonamides substitués en position 5 et répondant à la formule générale III:

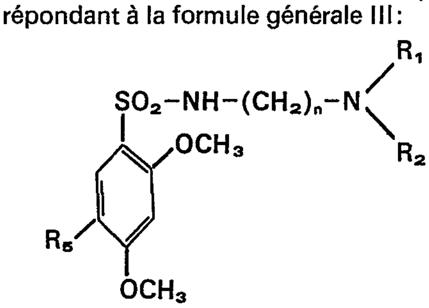

(III)

dans laquelle n, $R_1$, $R_2$ et $R_5$ ont les significations ci-dessus indiquées.

Une classe avantageuse de composés selon l'invention est constituée par les diméthoxy-(2,4)-benzènesulfonamides de formule III dans laquelle

n, $R_1$ et $R_2$ ont les significations ci-dessus indiquées et $R_5$ représente l'hydrogène. Ces composés sont disubstitués.

Parmi ces composés disubstitués, une classe avantageuse de composés conformes à l'invention est constituée par les composés de formule III dans laquelle:

n vaut 2 ou 3;

$R_5$ représente un atome d'hydrogène, et

N—$R_1$ représente l'un des radicaux choisis
$R_2$
dans le groupe comprenant les radicaux diméthylamino, diéthylamino, pyrrolidino, pipéridino, morpholino et pipéridino substitué par un groupe méthyle, notamment en position 2.

Une classe avantageuse de composés conformes à l'invention est constituée par les diméthoxy-(2,4)-benzènesulfonamides substitués en position 5, de formule III dans laquelle n, $R_1$ et $R_2$ ont les significations ci-dessus indiquées, $R_5$ a également la signification ci-dessus indiquée, à l'exception de l'hydrogène. Ces composés sont trisubstitués.

Parmi ces composés trisubstitués, une classe avantageuse de composés conformes à l'invention est constituée par les composés de formule III, dans laquelle $R_5$ représente Cl, Br, $OCH_3$, $SO_2C_2H_5$, $SO_2nC_3H_7$ ou $SO_2iC_3H_7$.

Une autre classe avantageuse de composés trisubstitués conformes à l'invention est constituée par les composés de formule III dans laquelle $R_5$ représente Cl, Br ou $OCH_3$.

Une autre classe de composés trisubstitués préférés selon l'invention est constituée par les composés de formule III dans laquelle:

n vaut 2 ou 3;

$R_5$ représente le groupe $OCH_3$, Cl, Br, $SO_2-CH_3$, $SO_2C_2H_5$, $SO_2nC_3H_7$ ou $SO_2iC_3H_7$, et

N—$R_1$ représente l'un des radicaux choisis
$R_2$
dans le groupe comprenant les radicaux diméthylamino, diéthylamino, pyrrolidino, pipéridino, morpholino et pipéridino substitué par un groupe méthyle, notamment en position 2.

Une classe préférée de composés trisubstitués conformes à l'invention est constituée par les composés de formule III dans laquelle:

n vaut 2 ou 3;

$R_5$ représente Cl, Br, $OCH_3$, $SO_2CH_3$ ou $SO_2C_2H_5$, et

N—$R_1$ représente le radical pyrrolidino, pipéri-
$R_2$
dino ou morpholino.

Une classe particulièrement avantageuse de composés trisubstitués est constituée par les composés de formule III dans laquelle:

n vaut 2 ou 3;

$R_5$ représente Cl, Br ou $SO_2CH_3$, et

N—$R_1$ représente le radical pyrrolidino, pipéri-
$R_2$
dino ou morpholino.

Une autre classe avantageuse de composés trisubstitués conformes à l'invention est constituée par les composés de formule III dans laquelle:

n vaut 3;

$R_5$ représente Cl, Br, $SO_2CH_3$ ou $OCH_3$, et

N—$R_1$ représente le radical morpholino.
$R_2$

Une autre classe avantageuse de composés trisubstitués conformes à l'invention est constituée par les composés de formule III dans laquelle:

n vaut 2;

$R_5$ représente $SO_2C_2H_5$, et

N—$R_1$ représente le radical diéthylamino; ou
$R_2$

n vaut 3;

$R_5$ représente $SO_2C_2H_5$, et

N—$R_1$ représente le radical diméthylamino ou
$R_2$
diéthylamino.

Les composés de ces divers groupes préférés sont avantageusement sous forme de sels, notamment de chlorhydrate.

Pour former les composés selon l'invention, on peut avoir recours aux sulfohalogénures, notamment aux sulfochlorures, de formule IV:

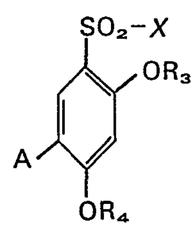

(IV)

dans laquelle:

X est un groupe halogène, notamment brome ou de préférence chlore;

$R_3$ et $R_4$ représentent chacun, indépendamment l'un de l'autre, un radical alcoyle inférieur ayant de 1 à 4 atomes de carbone, et

A a l'une quelconque des significations indiquées ci-dessus pour $R_5$, à l'exception de $NH_2$ (c'est-à-dire représente l'hydrogène, un halogène, les radicaux alcoxy ayant de 1 à 4 atomes de carbone, les groupes alcoylsulfonyle ayant de 1 à 4 atomes de carbone, le groupe $NO_2$ ou $CF_3$).

Pour préparer les sulfohalogénures de formule IV ci-dessus indiquée, notamment les sulfochlorures de formule V:

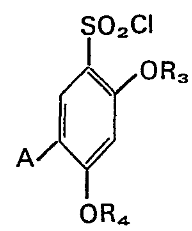

(V)

dans laquelle A, $R_3$ et $R_4$ ont les significations indiquées ci-dessus, et on peut avoir recours à l'arylamine de formule VI:

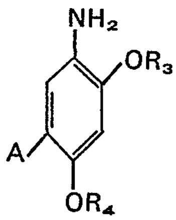

(VI)

dans laquelle A, $R_3$ et $R_4$ ont les significations indiquées ci-dessus, et à partir de laquelle:

a) on forme le sel de diazonium, notamment le chlorure de diazonium de formule VII. Ce sel de diazonium est notamment obtenu en faisant réagir l'arylamine au sein d'une solution de l'acide halogéné correspondant, notamment d'acide chlorhydrique, avec une solution de nitrite d'un métal alcalin, et en maintenant le mélange réactionnel à une température de préférence inférieure à environ 10°C;

b) on fait ensuite réagir le sel de diazonium obtenu en solution avec de l'anhydride sulfureux. On opère de préférence en présence d'acide acétique, ainsi que d'un catalyseur apte à contribuer à la transformation du groupe diazonium en groupe sulfohalogénure, notamment sulfochlorure. Ce catalyseur est, par exemple, à base de cuivre (réaction de Sandmeyer modifiée).

Les étapes a et b de cette réaction, appliquées à titre d'exemple à la préparation des sulfochlorures de formule V, peuvent être représentées comme suit:

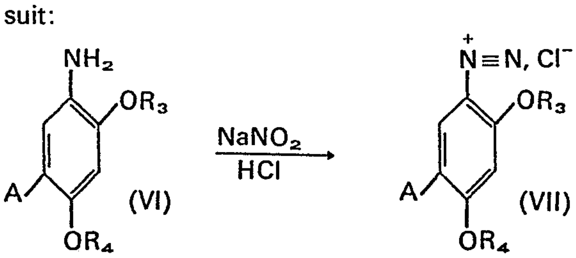


(VI) (VII)

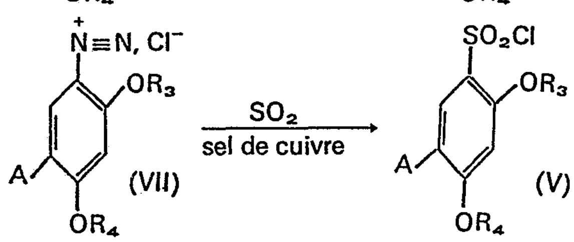


(VII) (V)

A titre d'arylamines préférés de la préparation des sulfochlorures de formule V on peut citer:

la diméthoxy-2,4 aniline,
la chloro-5-diméthoxy-2,4-aniline,
la bromo-5-diméthoxy-2,4-aniline,
la triméthoxy-2,4,5-aniline,
la diméthoxy-2,4-méthylsulfonyl-5-aniline,
la diméthoxy-2,4-éthylsulfonyl-5-aniline,
la diméthoxy-2,4-propylsulfonyl-5-aniline,
la diméthoxy-2,4-isopropylsulfonyl-5-aniline.

Pour préparer les sulfochlorures de formule V dans laquelle A a la signification ci-dessus indiquée, on peut également procéder par sulfonation ou halogénosulfonation, de préférence chlorosulfonation du composé de formule VIII dans laquelle A, $R_3$ et $R_4$ ont les significations sus-indiquées:

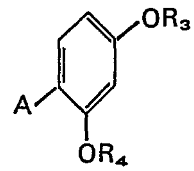

(VIII)

Dans le cas de la sulfonation (par réaction du composé VIII avec de l'acide sulfurique), on obtient, dans une première étape, l'acide sulfonique de formule IX:

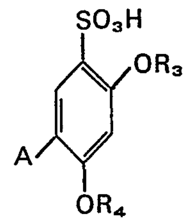

(IX)

L'acide IX obtenu peut ensuite être transformé en sel d'une base organique ou minérale, par action de la base appropriée telle que l'hydroxyde de sodium, l'hydroxyde de potassium ou la pyridine.

Ce sel est représenté par la formule X suivante:

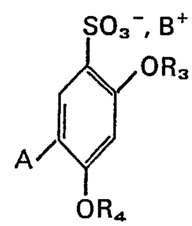

(X)

dans laquelle $B^+$ représente un métal, notamment alcalin ou alcalino-terreux, et A a l'une quelconque des significations indiquées ci-dessus. Le chlorure de formule V est alors obtenu par action sur le composé de formule IX – ou sur un sel organique ou minéral correspondant de formule X – d'un agent chlorant, tel que le chlorure de thionyle, le pentachlorure de phosphore ou l'oxychlorure de phosphore.

A titre d'exemple, le schéma réactionnel de l'obtention des composés dans lesquels A a l'une quelconque des signfiications indiquées ci-dessus, à partir des composés de formule VIII, peut se représenter comme suit:

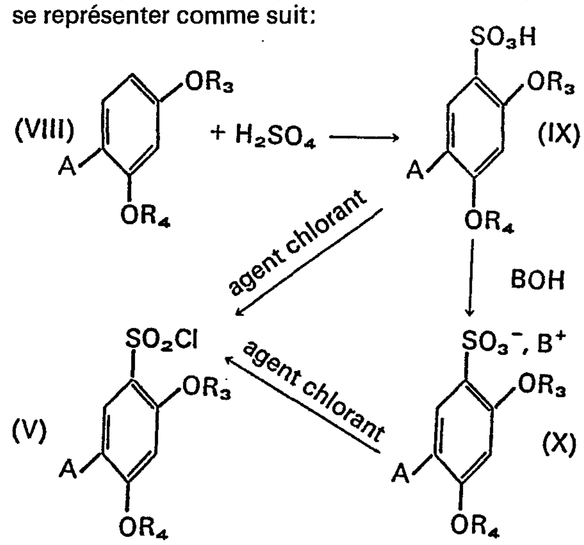


(VIII) (IX) (V) (X)

Dans le cas de la chlorosulfonation, on fait réagir l'acide chlorosulfonique sur le composé de formule VII dans laquelle A, $R_3$ et $R_4$ ont les significations ci-dessus indiquées.

La réaction peut s'écrire:

(VIII) 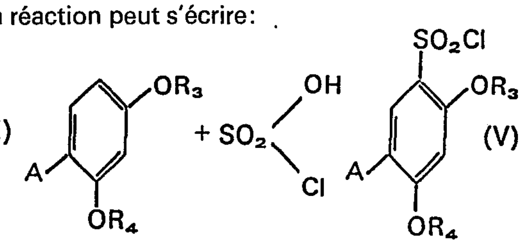 (V)

Le chlorhydrate des composés de formule I peut être obtenu par réaction d'une amine de formule XI:

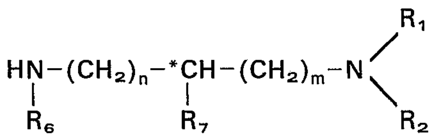 (XI)

dans laquelle:

n et m, indépendamment l'un de l'autre, prennent les valeurs de 0 à 4, et

$R_1$, $R_2$, $R_6$ et $R_7$ ont les significations ci-dessus indiquées sur un sulfochlorure de formule V:

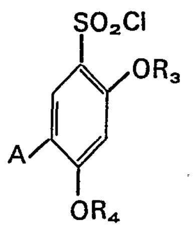 (V)

dans laquelle:

$R_3$ et $R_4$ ont les significations ci-dessus indiquées, et

A a l'une quelconque des significations ci-dessus indiquées pour $R_5$, à l'exception du groupe $NH_2$ (étant entendu que l'on obtiendra un autre halogénate des composés de formule I si l'on met en œuvre un autre sulfohalogénure, par exemple un sulfobromure, en lieu et place dudit sulfochlorure).

L'obtention des composés de formule I dans laquelle $R_5$ représente $NH_2$ est réalisée en réduisant le composé de formule I dans lequel $R_5$ représente $NO_2$ par hydrogénation catalytique ou par réduction chimique.

Le chlorhydrate des composés de formule I obtenus comme il vient d'être indiqué a pour formule XII:

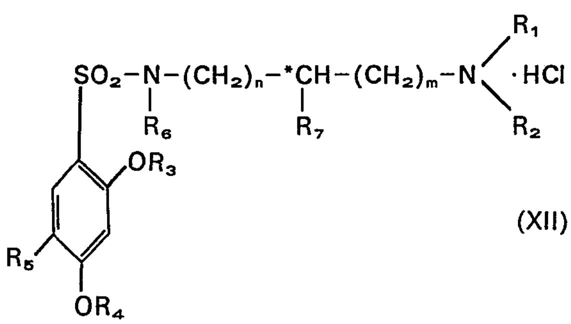 (XII)

Le passage du chlorhydrate de formule XII ci-dessus indiquée au composé de formule I, non salifié, c'est-à-dire sous forme de base, peut se faire en solution, par réaction avec une base forte telle que l'hydroxyde de sodium ou de potassium, ou toute autre base présentant des caractères de basicité équivalents.

La transformation du chlorhydrate de formule XII en un sel différent peut se faire en passant par la base de formule I, puis en salifiant celle-ci selon des procédés classiques.

Un groupe d'amines préférés utilisées dans la préparation des composés de formule I, ou de leurs sels correspondants, est constitué par les suivantes:

la diméthylaminoéthylamine,
la diéthylaminoéthylamine,
la pyrrolidinoéthylamine,
la pipéridinoéthylamine,
la morpholinoéthylamine,
la diméthylaminopropylamine,
la diéthylaminopropylamine,
la pipéridinopropylamine,
la (méthyl-2-pipéridino)-3-propylamine,
la morpholinopropylamine.

Les exemples suivants, relatifs à la préparation d'un certain nombre de sulfochlorures et de nouveaux benzènesulfonamides, servent à illustrer l'invention mais ne sont pas limitatifs.

*Exemple 1:*

*Préparation du chlorure de diméthoxy-2,4-benzènesulfonyle*

Elle s'effectue en deux étapes, en suivant la technique de C.M. Suter et H.L. Hansen rapportée dans «J. of Am. Chem. Soc.» 1933, *55*, 2080.

*1^re^ étape:*

*Obtention du diméthoxy-2,4-benzènesulfonate de potassium*

Dans un tricol de 250 $cm^3$, muni d'un thermomètre, d'une ampoule à réactif et d'un système d'agitation magnétique, sont placés 85 g (0,615 mol) de diméthoxy-1,3-benzène. Après refroidissement vers 0°C sont ajoutés goutte à goutte 50 $cm^3$ (0,9 mol) d'acide sulfurique (d = 1,83-1,84). Le mélange est ensuite ramené à la température ambiante et laissé en contact 1,5 h. Le milieu réactionnel pris en masse est versé dans 750 $cm^3$ d'une solution saturée de carbonate de potassium (138 g/l), puis est mis au repos une nuit. Le précipité obtenu est filtré puis séché sous vide phosphorique.

Rendement 79%.

*2^e^ étape:*

*Passage au chlorure de diméthoxy-2,4-benzènesulfonyle*

Dans un tricol de 1 l, muni d'un système d'agitation et d'une garde à chlorure de calcium, sont introduits successivement 124 g (0,484 mol) de diméthoxy-2,4-benzènesulfonate de potassium et 500 $cm^3$ de diméthylformamide. A la suspension obtenue sont ajoutés goutte à goutte

et à température ambiante 69,5 g (0,583 mol) de chlorure de thionyle. Après une nuit de contact, le mélange est versé sur de la glace pilée. Le précipité blanc obtenu est essoré, lavé abondamment à l'eau, puis séché sous vide phosphorique.

Rendement 75%; F 72°C (litt. 70,5°C).

*Exemple 2:*

*Préparation du chlorure de triméthoxy-2,4,5-benzènesulfonyle*

Elle peut être effectuée selon deux procédés.

*1er procédé:*

Dans un tricol de 500 cm³, muni d'un thermomètre, d'une ampoule à réactif, d'une garde à chlorure de calcium et d'un système d'agitation magnétique, sont placés 42 g (0,25 mol) de triméthoxy-1,2,4-benzène en solution dans 200 cm³ de chloroforme pur. Au milieu réactionnel, placé sous atmosphère d'azote et refroidi vers −10°C, sont ajoutés goutte à goutte 80 cm³ d'acide chlorosulfonique. Il se forme successivement un précipité laiteux blanc crème, puis une solution verdâtre qui vire au brun. L'addition terminée, le milieu réactionnel est laissé en contact 1 h à température ambiante, puis est versé sur de la glace pilée. Le précipité obtenu est extrait par du chloroforme; la phase organique est ensuite séchée sur sulfate de sodium, puis évaporée sous pression réduite. Le résidu brun formé est lavé avec le minimum de toluène jusqu'à l'obtention d'un solide beige qui est ensuite chromatographié sur colonne de silice.

L'élution avec du benzène, puis avec un mélange benzène/chloroforme (50/50), donne le chlorure de triméthoxy-2,4,5-benzènesulfonyle.

Rendement 46%; F 147°C.

RMN ($CDCl_3$) à 80 MHz: δ 7,29 ppm (s, 1H, ArH); δ 6,55 ppm (s, 1H, ArH); δ 3,93, 3,78 et 3,53 ppm (3s, 9H, $OCH_3$).

IR (KBr): γ $SO_2$, as 1350 $cm^{-1}$, s 1160 $cm^{-1}$.

*2e procédé:*

Il comporte quatre étapes:

*Diméthoxy-3,4-chlorobenzène*

Dans un tricol de 1 l, muni d'un système d'agitation magnétique, d'un thermomètre, d'une garde à chlorure de calcium et d'une ampoule à réactif, sont introduits à froid vers 0°C et successivement 138 g (1 mol) de vératrole, puis goutte à goutte 135 g (1 ml) de chlorure de sulfuryle. L'addition terminée, le milieu réactionnel est ramené à température ambiante puis, après un contact de 1 h, est distillé sous pression réduite.

Rendement 83%, PE 120°C sous 1999,83 Pa (15 mmHg).

*Diméthoxy-4,5-nitro-2-chlorobenzène*

Dans un tricol de 1 l, muni d'un système d'agiation magnétique, d'un thermomètre et d'une ampoule à réactif, sont introduits successivement 143,9 g (0,83 mol) de diméthoxy-3,4-chlorobenzène, puis goutte à goutte 166 cm³ d'acide nitrique (d = 1,38), sans que la température dépasse 25°C. L'addition terminée, le mélange réactionnel est laissé 1,5 h en contact puis filtré.

Rendement 95%; F 105°C.

*Triméthoxy-2,4,5 nitrobenzène*

Dans un ballon de 2 l, muni d'un réfrigérant, sont introduits successivement une solution de potasse méthanolique (100 g de KOH dans 500 cm³ de méthanol), puis 100 g (0,46 ml) de diméthoxy-4,5-nitro-2-chlorobenzène et du carborandum. Le mélange est porté à l'ébullition à reflux 6 h. Après refroidissement, le milieu réactionnel est filtré; le précipité obtenu est lavé avec du méthanol.

Rendement 95%; F 129°C.

*Triméthoxy-2,4,5-aniline*

Dans un ballon de 500 cm³, muni d'un réfrigérant, sont ajoutés successivement 21,3 g (0,1 mol) de triméthoxy-2,4,5-nitrobenzène, 80 g de chlorure stanneux chimiquement pur pour miroitiers, 100 cm³ d'une solution d'acide chlorhydrique (d = 1,18) et du carborandum. Le mélange est porté à l'ébullition à reflux 1 h. Après refroidissement, une solution de lessive de soude est ajoutée jusqu'à dissolution du précipité. La solution obtenue est extraite par du chlorure de méthylène. Les extraits organiques sont séchés sur sulfate de sodium, puis évaporés sous pression réduite. Le résidu obtenu est cristallisé dans de l'éthanol.

Rendement 80%; F 94°C.

*Chlorure de triméthoxy-2,4,5-benzènesulfonyle*

Dans un tricol de 250 cm³, muni d'un système d'agitation et d'un thermomètre, sont introduits successivement 18,3 g (0,1 mol) de triméthoxy-2,4,5-aniline, puis 50 cm³ d'une solution d'acide chlorhydrique (d = 1,18). Après un contact de 4 h, l'amine est diazotée à −5°C par addition d'une solution de nitrite de sodium (10 g de $NaNO_2$ dans 50 cm³ d'eau). Le sel de diazonium obtenu est versé lentement dans un tricol, chauffé à 40°C et sous atmosphère d'azote, contenant 200 cm³ d'acide acétique saturé en anhydride sulfureux et 7 g de chlorure cuivrique. Le mélange est porté 2 h à 60°C, puis est versé sur de la glace pilée.

Rendement 35%; F 147°C.

*Exemple 3:*

*Préparation du chlorure de chloro-5-diméthoxy-2,4-benzènesulfonyle*

Elle est effectuée en deux étapes à partir du diméthoxy-1,3-benzène.

*1re étape:*

*Obtention du diméthoxy-2,4 chlorobenzène*

Elle s'effectue en suivant la technique de G. Castelfranchi et G. Borra rapportée dans «Annali di Chimica» 1953, *43*, 293.

Dans un tricol de 500 cm³, muni d'un système d'agitation magnétique, d'un thermomètre, d'une garde à chlorure de calcium et d'une ampoule à réactif, et refroidi vers 10°C, sont introduits successivement 97,5 g (0,70 mol) de diméthoxy-1,3-benzène, puis goutte à goutte 96,5 g (0,70 mol) de chlorure de sulfuryle. Une fois l'addition terminée, la solution est ramenée à la température ambiante et laissée en contact 2 h, puis distillée.

Rendement 85%; PE 137°C sous 2399,80 Pa (18 mmHg).

*2e étape:*

*Passage au chlorure de chloro-5-diméthoxy-2,4-benzènesulfonyle*

Dans un tricol de 500 $cm^3$, muni d'un système d'agitation magnétique, d'une garde à chlorure de calcium, d'un thermomètre et d'une ampoule à réactif, sont introduits 35 g (0,2 mol) de diméthoxy-2,4-chlorobenzène en solution dans 250 $cm^3$ de chloroforme pur. La solution est refroidie vers 0°C, puis est additionnée goutte à goutte de 50 $cm^3$ (0,75 mol) d'acide chlorosulfonique. Une fois l'addition terminée, le milieu réactionnel est ramené à température ambiante, puis laissé en contact 3 h; il est ensuite versé sur de la glace pilée. Le mélange obtenu est épuisé au chloroforme. Les extraits organiques sont séchés sur sulfate de sodium, puis concentrés jusqu'à cristallisation. Le solide obtenu est recristallisé dans un mélange éther éthylique/benzène.

Rendement 74%; F 175°C.

RMN ($CDCl_3$) à 80 MHz: δ 7,87 ppm (s, 1H, ArH); δ 6,55 ppm (s, 1H, ArH); δ 4 et 3,96 ppm (2s, 6H, $OCH_3$).

IR (KBr): γ $SO_2$, as 1385 $cm^{-1}$, s 1170 $cm^{-1}$.

*Exemple 4:*

*Préparation du chlorure de bromo-5-diméthoxy-2,4-benzènesulfonyle*

Elle est effectuée en deux étapes à partir du diméthoxy-1,3-benzène.

*1re étape:*

*Obtention du diméthoxy-2,4-bromobenzène*

Elle s'effectue en suivant le protocole de S.T. Feng et K.Y. Chiu rapportée dans «Hsûeh Pao», 1959, *25*, 277.

Dans un tricol de 250 $cm^3$, muni d'un système d'agitation magnétique, d'un thermomètre, et refroidi vers 10°C, sont ajoutés successivement 27,6 g (0,2 mol) de diméthoxy-1,3-benzène, puis par petites portions 36 g (0,2 mol) de N-bromosuccinimide. Une fois l'addition terminée, le milieu réactionnel est ramené à température ambiante et laissé en contact 2 h. Après lavage à l'eau et extraction au chloroforme, les extraits organiques sont séchés sur sulfate de sodium, puis concentrés sous pression réduite. Le liquide résiduel est distillé.

Rendement 82%; PE 1950°C sous 199,83 Pa (15 mmHg).

*2e étape:*

*Passage au chlorure de bromo-5-diméthoxy-2,4-benzènesulfonyle*

Il est obtenu selon le même protocole que celui décrit dans l'exemple 3 pour préparer le chlorure de chloro-5-diméthoxy-2,4-benzènesulfonyle. En utilisant 65,1 g (0,3 ml) de diméthoxy-2,4-bromobenzène et 100 $cm^3$ d'acide chlorosulfonique.

Rendement 77%; F 195°C.

RMN ($CDCl_3$) à 60 MHz: δ 7,85 ppm (s, 1H, ArH); δ 6,70 ppm (s, 1H, ArH); δ 3,92 et 3,88 ppm (2s, 6H, $OCH_3$).

IR (KBr): γ $SO_2$, as 1385 $cm^{-1}$, s 1165 $cm^{-1}$.

*Exemple 5:*

*Chorures d'alkylsulfonyl-5-diméthoxy-2,4-benzènesulfonyles*

Ils sont obtenus à partir du chlorure de diméthoxy-2,4-benzènesulfonyle décrit dans l'exemple 1. Le schéma général de leur préparation est le suivant:

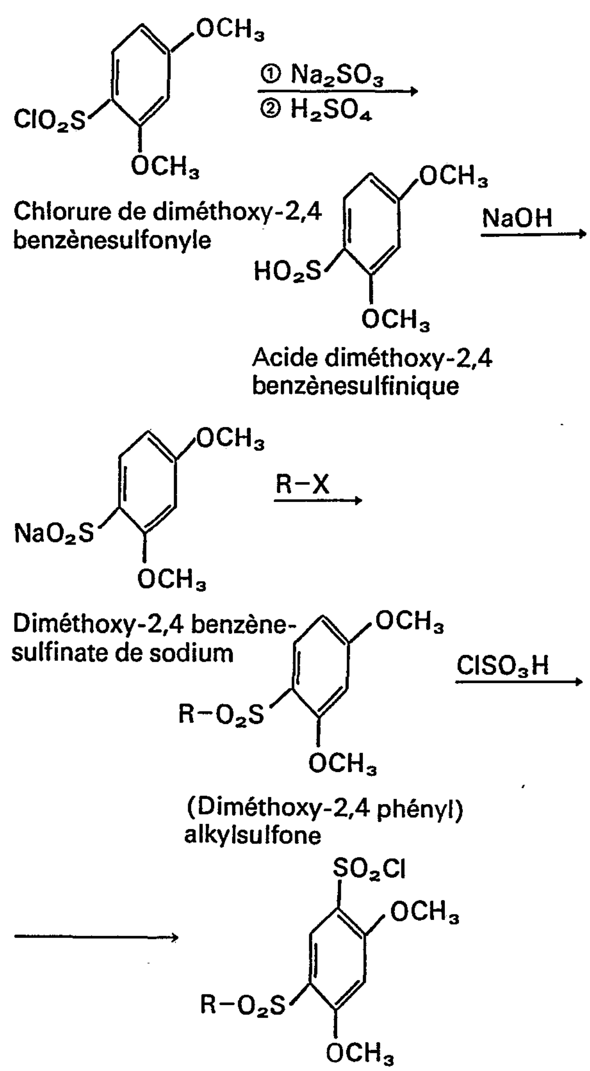


Chlorure de diméthoxy-2,4 benzènesulfonyle

Acide diméthoxy-2,4 benzènesulfinique

Diméthoxy-2,4 benzène-sulfinate de sodium

(Diméthoxy-2,4 phényl) alkylsulfone

Chlorure d'alkylsulfonyl-5 diméthoxy-2,4 benzènesulfonyle

*Acide diméthoxy-2,4-benzènesulfinique*

A une solution aqueuse contenant 129 g (0,974 mol) de sulfite de sodium sont ajoutés par petites portions et sous agitation 115 g (0,487 mol) de chlorure de diméthoxy-2,4-benzènesulfonyle. Durant cette période, le pH est maintenu alcalin par addition de lessive de soude. Après 3 h de contact, le milieu réactionnel est filtré; le filtrat est acidifié par de l'acide sulfurique 2N jusqu'à précipitation de l'acide sulfinique.

Rendement 72%; F 122°C.

*Diméthoxy-2,4-benzènesulfinate de sodium*

Il est obtenu par addition de la quantité stœchiométrique d'hydroxyde de sodium en solution dans l'eau. La solution de sulfinate est évaporée à sec.

*(Diméthoxy-2,4-phényl)alkylsulfones*

Protocole général:

Dans un ballon de 500 $cm^3$, muni d'un réfrigérant et d'un système d'agitation magnétique, sont ajoutés successivement 0,1 mol de diméthoxy-2,4-benzènesulfinate de sodium, 250 $cm^3$ d'isopropanol, puis 0,15 mol d'halogénure d'alkyle, de préférence un iodure. Le mélange est porté à l'ébullition à reflux 5 à 30 h selon l'halogénure mis en œuvre. Après refroidissement, le milieu réactionnel est évaporé à sec. Le résidu est repris par de l'eau, puis est extrait par du chloroforme. L'évaporation de la phase organique, après séchage sur sulfate de sodium, fournit une huile qui est cristallisée dans du benzène.

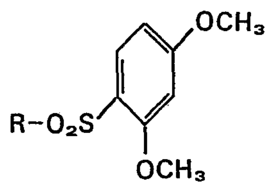

| R | PM | Temps de chauffage (h) | F (°C) | Rdt (%) |
|---|---|---|---|---|
| $CH_3$ | 216 | 5 | 109 | 65 |
| $C_2H_5$ | 230 | 7 | 94 | 70 |
| $nC_3H_7$ | 244 | 15 | 70 | 90 |
| $iC_3H_7$ | 244 | 30 | 100 | 64 |

*Chlorures d'alkylsulfonyl-5-diméthoxy-2,4-benzènesulfonyles*

Protocole général:

Dans un tricol de 1 l, muni d'un système d'agitation magnétique, d'une garde à chlorure de calcium, d'un thermomètre et d'une ampoule à réactif, est introduite une solution de (diméthoxy-2,4-phényl)alkylsulfone (0,2 mol) dans 200 $cm^3$ de chloroforme pur. Après refroidissement vers −10°C sont ajoutés goutte à goutte 100 $cm^3$ d'acide chlorosulfonique. Une fois l'addition terminée, le mélange est laissé en contact 0,5 h à −10°C, puis est ramené à température ambiante et laissé sous agitation pendant 7 h; il est ensuite versé sur de la glace pilée. Le mélange obtenu est extrait par du chloroforme. La phase organique est séchée sur sulfate de sodium, filtrée, puis évaporée sous pression réduite. Le résidu est cristallisé dans du benzène.

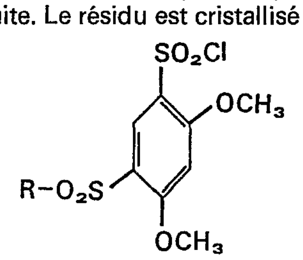

| R | PM | F (°C) | Rdt (%) |
|---|---|---|---|
| $CH_3$ | 314,5 | 204 | 52 |
| $C_2H_5$ | 328,5 | 191 | 77 |
| $nC_3H_7$ | 342,5 | 169 | 87 |
| $iC_3H_7$ | 342,5 | 218 | 66 |

*Chlorure de diméthoxy-2,4-méthylsulfonyl-5-benzènesulfonyle*

RMN (DMSO) à 80 MHz: δ 8,2 ppm (s, 1H, ArH); δ 6,9 ppm (s, 1H, ArH); δ 4,1 et 4,05 ppm (2s, 6H, $OCH_3$); δ 3,2 ppm (s, 3H, $CH_3$).

IR (KBr): γ $\underline{SO_2}-Cl$, as 1360 $cm^{-1}$, s 1170 $cm^{-1}$; γ $\underline{SO_2}-CH_3$, as 1300 $cm^{-1}$, s 1140 $cm^{-1}$.

*Chlorure d'éthylsulfonyl-5-diméthoxy-2,4-benzènesulfonyle*

RMN (DMSO) à 80 MHz: δ 8,2 ppm (s, 1H, ArH); δ 6,93 ppm (s, 1H, ArH); δ 4,15 et 4,1 ppm (2s, 6H, $OCH_3$); δ 3,25 ppm (q, 2H, $-CH_2-$); δ 1,18 ppm (t, 3H, $CH_3-$).

IR (KBr): γ $\underline{SO_2}-Cl$, as 1370 $cm^{-1}$, s 1175 $cm^{-1}$; γ $\underline{SO_2}-CH_2-$, as 1315 $cm^{-1}$, s 1140 $cm^{-1}$.

*Chlorure de diméthoxy-2,4-propylsulfonyl-5-benzènesulfonyle*

RMN (DMSO) à 80 MHz: δ 8,05 ppm (s, 1H, ArH); δ 6,72 ppm (s, 1H, ArH); δ 3,92 et 3,87 ppm (2s, 6H, $OCH_3$); δ 3,2 ppm (t, 2H $-\underline{CH_2}-SO_2-$); δ 1,45 ppm (m, 2H, $-\underline{CH_2}$); δ 0,85 ppm (t, 3H, $CH_3$).

IR (KBr): γ $\underline{SO_2}Cl$, as 1370 $cm^{-1}$, s 1180 $cm^{-1}$; γ $\underline{SO_2}-CH_2$, as 1315 $cm^{-1}$, s 1135 $cm^{-1}$.

*Chlorure d'isopropylsulfonyl-5-diméthoxy-2,4-benzènesulfonyle*

RMN (DMSO) à 80 MHz: δ 8,02 ppm (s, 1H, ArH); δ 6,7 ppm (s, 1H, ArH); δ 3,92 et 3,85 ppm (2s, 6H, $OCH_3$); δ 3,48 ppm (m, 1H, $-\underset{|}{C}H-$); δ 1,11 et 1,03 ppm (2s, 6H, $CH_3$).

IR (KBr): γ $\underline{SO_2}-Cl$, as 1365 $cm^{-1}$, s 1175 $cm^{-1}$; γ $\underline{SO_2}-CH<$, as 1300 $cm^{-1}$, s 1130 $cm^{-1}$.

*Exemple 6:*

*Préparation des diméthoxy-2,4-benzènesulfonamides N-substitués*

A 0,015 mol de chlorure de benzènesulfonyle en solution dans un mélange de chlorure de méthylène (40 $cm^3$) et de méthanol (10 $cm^3$) est ajouté 0,015 mol de dialkylaminoalkylamine en solution dans 10 $cm^3$ de chlorure de méthylène. Après un contact de 2 h, sous agitation et à température ambiante, le milieu réactionnel est évaporé à sec puis est repris par 50 $cm^3$ d'eau. La solution aqueuse est lavée par de l'éther éthylique, puis évaporée à sec. Le résidu est cristallisé dans un mélange isopropanol/méthanol. Le passage éventuel à la base s'effectue en traitant la solution aqueuse du chlorhydrate par de la soude N jusqu'à précipitation. Le précipité est extrait par du chlorure de méthylène. La solution organique est séchée, puis évaporée sous pression réduite. La base obtenue est cristalli-

sée dans un mélange éther éthylique/éther de pétrole.

D'autres composés ont été préparés de façon analogue et sont rassemblés dans le tableau I (le point de fusion indiqué pour chaque composé est, sauf indication contraire, celui du chlorhydrate).

*Tableau I*

*Composés de formule:*

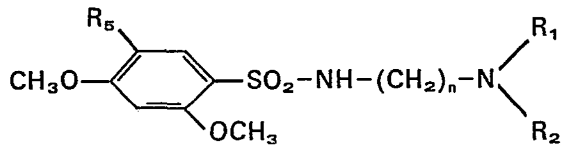

| Produit N° | 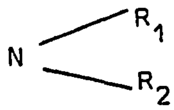 | n | $R_5$ | Formule du composé sous forme de chlorhydrate | Poids moléculaire | Point de fusion du chlorhydrate | Rdt (%) |
|---|---|---|---|---|---|---|---|
| 880 | 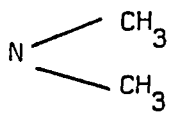 | 2 | Cl | $C_{12}H_{20}Cl_2N_2O_4S$ | 359 | 237 | 78 |
| 881 | 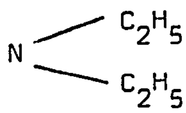 | 2 | Cl | $C_{14}H_{24}Cl_2N_2O_4S$ | 387 | 202 | 93 |
| 882 | 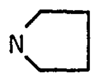 | 2 | Cl | $C_{14}H_{22}Cl_2N_2O_4S$ | 385 | 209 | 83 |
| 883 | 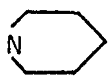 | 2 | Cl | $C_{15}H_{24}Cl_2N_2O_4S$ | 399 | 220 | 83 |
| 884 | 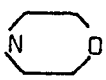 | 2 | Cl | $C_{14}H_{22}Cl_2N_2O_5S$ | 401 | 232 | 82 |
| 886 | 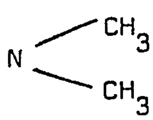 | 3 | Cl | $C_{13}H_{22}Cl_2N_2O_4S$ | 373 | 203 | 89 |
| 887 | 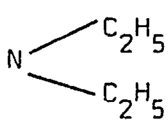 | 3 | Cl | $C_{15}H_{26}Cl_2N_2O_4S$ | 401 | 179 | 60 |
| 888 | 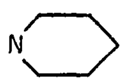 | 3 | Cl | $C_{16}H_{26}Cl_2N_2O_4S$ | 413 | 191 | 85 |
| 889 | 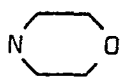 | 3 | Cl | $C_{15}H_{24}Cl_2N_2O_5S$ | 415 | 212 | 61 |
| 897 | 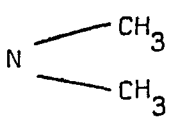 | 2 | $OCH_3$ | $C_{13}H_{23}ClN_2O_5S$ | 354,5 | 247 | 90 |
| 898 | 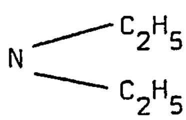 | 2 | $OCH_3$ | $C_{15}H_{27}ClN_2O_5S$ | 382,5 | 194 | 76 |

*Tableau I* (suite)

| Produit N° | 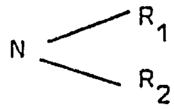 | n | $R_5$ | Formule du composé sous forme de chlorhydrate | Poids moléculaire | Point de fusion du chlorhydrate | Rdt (%) |
|---|---|---|---|---|---|---|---|
| 899 | 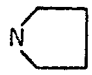 | 2 | $OCH_3$ | $C_{15}H_{25}ClN_2O_5S$ | 380,5 | 186 | 89 |
| 900 | 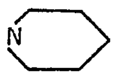 | 2 | $OCH_3$ | $C_{16}H_{27}ClN_2O_5S$ | 394,5 | 207 | 66 |
| 901 | 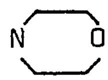 | 2 | $OCH_3$ | $C_{15}H_{25}ClN_2O_6S$ | 396,5 | 220 | 48 |
| 893 | 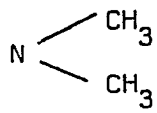 | 3 | $OCH_3$ | $C_{14}H_{25}ClN_2O_5S$ | 368,5 | 212 | 57 |
| 891 | 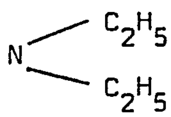 | 3 | $OCH_3$ | $C_{16}H_{29}ClN_2O_5S$ | 396,5 | 173 | 86 |
| 894 | 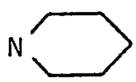 | 3 | $OCH_3$ | $C_{17}H_{29}ClN_2O_5S$ | 408,5 | 236 | 56 |
| 895 | 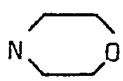 | 3 | $OCH_3$ | $C_{16}H_{27}ClN_2O_6S$ | 410,5 | 225 | 51 |
| 924 | 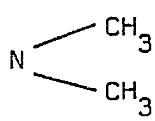 | 2 | Br | $C_{12}H_{20}BrClN_2O_4S$ | 403,71 | 244 | 49 |
| 925 | 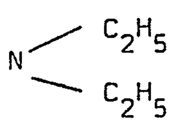 | 2 | Br | $C_{14}H_{24}BrClN_2O_4S$ | 431,77 | 202 | 48 |
| 926 | 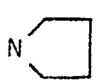 | 2 | Br | $C_{14}H_{22}BrClN_2O_4S$ | 429,75 | 226 | 64 |
| 927 | 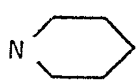 | 2 | Br | $C_{15}H_{24}BrClN_2O_4S$ | 443,78 | 204 | 71 |
| 928 | 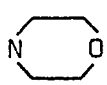 | 2 | Br | $C_{14}H_{22}BrClN_2O_5S$ | 445,75 | 246 | 71 |
| 930 | 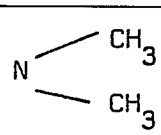 | 3 | Br | $C_{13}H_{22}BrClN_2O_4S$ | 417,74 | 227 | 85 |
| 931 | 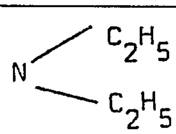 | 3 | Br | $C_{15}H_{26}BrClN_2O_4S$ | 445,79 | 190 | 60 |

*Tableau I* (suite)

| Produit N° | 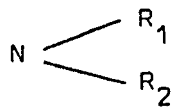 | n | $R_5$ | Formule du composé sous forme de chlorhydrate | Poids moléculaire | Point de fusion du chlorhydrate | Rdt (%) |
|---|---|---|---|---|---|---|---|
| 932 | 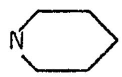 | 3 | Br | $C_{16}H_{26}BrClN_2O_4S$ | 457,80 | 200 | 60 |
| 933 | 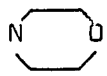 | 3 | Br | HCl, $C_{15}H_{24}BrClN_2O_5S$<br>base $C_{14}H_{23}BrN_2O_5S$ | 459,77 | 237<br>127 | 72 |
| 955 | 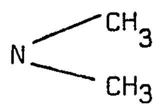 | 2 | $SO_2CH_3$ | $C_{13}H_{23}N_2O_6S_2Cl$ | 402,897 | 233 | 63 |
| 956 | 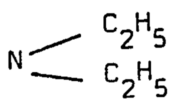 | 2 | $SO_2CH_3$ | $C_{15}H_{27}N_2O_6S_2Cl$ | 430,951 | 211 | 70 |
| 957 | 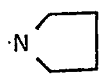 | 2 | $SO_2CH_3$ | $C_{15}H_{25}N_2O_6S_2Cl$ | 428,935 | 232 | 62 |
| 958 | 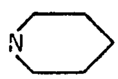 | 2 | $SO_2CH_3$ | $C_{16}H_{27}N_2O_6S_2Cl$ | 442,962 | 248 | 60 |
| 959 | 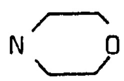 | 2 | $SO_2CH_3$ | $C_{15}H_{25}N_2O_7S_2Cl$ | 444,932 | 246 | 66 |
| 961 | 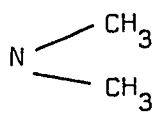 | 3 | $SO_2CH_3$ | $C_{14}H_{25}N_2O_6S_2Cl$ | 416,924 | 208 | 58 |
| 962 | 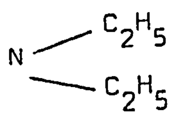 | 3 | $SO_2CH_3$ | $C_{16}H_{29}N_2O_6S_2Cl$ | 444,978 | 220 | 65 |
| 963 | 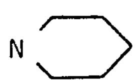 | 3 | $SO_2CH_3$ | $C_{17}H_{29}N_2O_6S_2Cl$ | 456,989 | 250 | 58 |
| 964 | 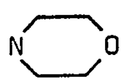 | 3 | $SO_2CH_3$ | $C_{16}H_{27}N_2O_7S_2Cl$ | 458,959 | 237 | 64 |
| 979 | 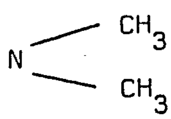 | 2 | $SO_2C_2H_5$ | $C_{14}H_{25}ClN_2O_6S_2$ | 416,924 | 235 | 72 |
| 980 | 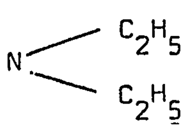 | 2 | $SO_2C_2H_5$ | $C_{16}H_{29}ClN_2O_6S_2$ | 444,978 | 242 | 71 |
| 981 | 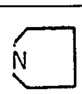 | 2 | $SO_2C_2H_5$ | $C_{16}H_{27}ClN_2O_6S_2$ | 442,962 | 246 | 73 |

*Tableau I* (suite)

| Produit N° | 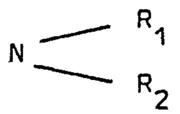 | n | $R_5$ | Formule du composé sous forme de chlorhydrate | Poids moléculaire | Point de fusion du chlorhydrate | Rdt (%) |
|---|---|---|---|---|---|---|---|
| 982 | 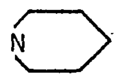 | 2 | $SO_2C_2H_5$ | $C_{17}H_{29}ClN_2O_6S_2$ | 456,989 | 247 | 63 |
| 983 | 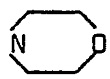 | 2 | $SO_2C_2H_5$ | $C_{16}H_{27}ClN_2O_7S_2$ | 458,959 | 243 | 44 |
| 985 | 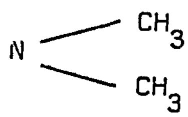 | 3 | $SO_2C_2H_5$ | $C_{15}H_{26}N_2O_6S_2$* | 394,490* | 170* | 60 |
| 986 | 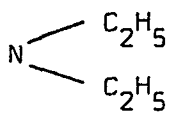 | 3 | $SO_2C_2H_5$ | $C_{17}H_{31}ClN_2O_6S_2$ | 459 | 176 | 80 |
| 987 | 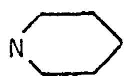 | 3 | $SO_2C_2H_5$ | $C_{18}H_{31}ClN_2O_6S_2$ | 471 | 203 | 42 |
| 988 | 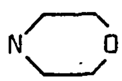 | 3 | $SO_2C_2H_5$ | $C_{17}H_{29}ClN_2O_7S_2$ | 472,986 | 204 | 70 |
| 1072 | 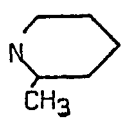 | 3 | $SO_2C_2H_5$ | $C_{19}H_{33}ClN_2O_6S$ | 485,043 | 170 | 83 |
| 1061 | 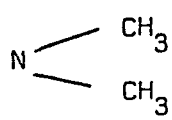 | 2 | H | $C_{12}H_{21}ClN_2O_4S$ | 324,82 | 204 | 72 |
| 1062 | 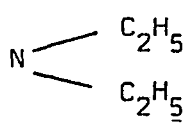 | 2 | H | $C_{14}H_{25}ClN_2O_4S$ | 352,87 | 154 | 69 |
| 1063 | 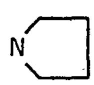 | 2 | H | $C_{14}H_{23}ClN_2O_4S$ | 350,85 | 125 | 87 |
| 1064 | 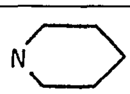 | 2 | H | HCl, $C_{15}H_{25}ClN_2O_4S$<br>base $C_{15}H_{24}N_2O_4S$ | 364,88<br>328,42 | 164<br>108 | 77 |
| 1065 | 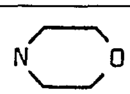 | 2 | H | HCl, $C_{14}H_{23}ClN_2O_5S$<br>base $C_{14}H_{22}N_2O_5S$ | 366,85<br>330,39 | 179<br>122 | 60 |
| 1067 | 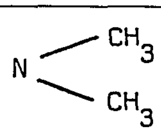 | 3 | H | HCl, $C_{13}H_{22}ClN_2O_4S$ | 337,83 | 157 | 65 |

* Les résultats concernent le composé sous forme de base.

*Tableau I* (suite)

| Produit N° | 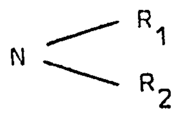 | n | $R_5$ | Formule du composé sous forme de chlorhydrate | Poids moléculaire | Point de fusion du chlorhydrate | Rdt (%) |
|---|---|---|---|---|---|---|---|
| 1068 | 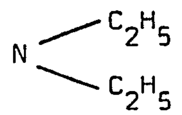 | 3 | H | HCl, $C_{15}H_{27}ClN_2O_4S$<br>base $C_{15}H_{26}N_2O_4S$ | 366,90<br>330,44 | 141<br>55 | 78 |
| 1069 | 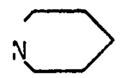 | 3 | H | HCl, $C_{16}H_{27}ClN_2O_4S$<br>base $C_{16}H_{26}N_2O_4S$ | 378,91<br>342,45 | 166<br>86 | 78 |
| 1070 | 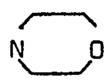 | 3 | H | $C_{15}H_{25}ClN_2O_5S$ | 380,88 | 195 | 45 |
| 1071 | 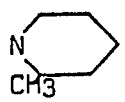 | 3 | H | $C_{17}H_{29}ClN_2O_4S$ | 392,94 | 176 | 50 |
| 1094 | 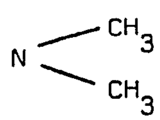 | 2 | $SO_2-nC_3H_7$ | $C_{15}H_{27}ClN_2O_6S_2$ | 430,95 | 163 | 73 |
| 1095 | 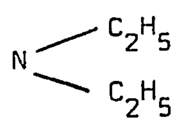 | 2 | $SO_2-nC_3H_7$ | $C_{17}H_{31}ClN_2O_6S_2$ | 459 | 224 | 91 |
| 1096 | 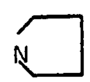 | 2 | $SO_2-nC_3H_7$ | $C_{17}H_{29}ClN_2O_6S_2$ | 456,99 | 206 | 71 |
| 1097 | 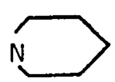 | 2 | $SO_2-nC_3H_7$ | $C_{18}H_{31}ClN_2O_6S_2$ | 471,02 | 226 | 89 |
| 1098 | 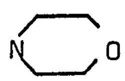 | 2 | $SO_2-nC_3H_7$ | $C_{17}H_{29}ClN_2O_7S_2$ | 472,99 | 186 | 83 |
| 1100* | 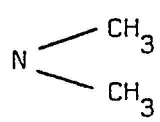 | 3 | $SO_2-nC_3H_7$ | $C_{16}H_{28}N_2O_6S_2$ base | 408,517 | 158* | 69 |
| 1101 | 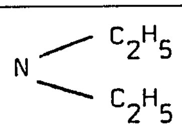 | 3 | $SO_2-nC_3H_7$ | $C_{18}H_{33}ClN_2O_6S_2$ | 473,03 | 158 | 65 |
| 1102* | 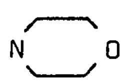 | 3 | $SO_2-nC_3H_7$ | $C_{18}H_{30}N_2O_7S_2$ base<br>HCl, $C_{18}H_{31}ClN_2O_7S_2$ | 450,55<br>487,01 | 122<br>150 | 68 |
| 1103 | 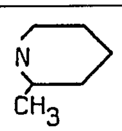 | 3 | $SO_2-nC_3H_7$ | $C_{20}H_{35}ClN_2O_6S_2$ | 499,07 | 167 | 71 |

* Les résultats concernent le composé sous forme de base.

*Tableau I* (suite)

| Produit N° | 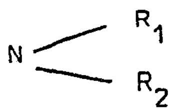 | n | $R_5$ | Formule du composé sous forme de chlorhydrate | Poids moléculaire | Point de fusion du chlorhydrate | Rdt (%) |
|---|---|---|---|---|---|---|---|
| 1104 | 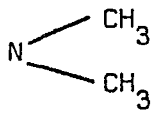 | 2 | $SO_2-iC_3H_7$ | HCl, $C_{15}H_{27}ClN_2O_6S_2$ | 430,95 | 217 | 71 |
| 1105 | 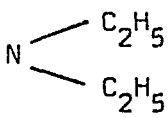 | 2 | $SO_2-iC_3H_7$ | $C_{17}H_{31}ClN_2O_6S_2$ | 459 | 201 | 84 |
| 1106 | 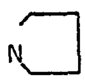 | 2 | $SO_2-iC_3H_7$ | $C_{17}H_{29}ClN_2O_6S_2$ | 456,99 | 222 | 77 |
| 1107 | 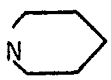 | 2 | $SO_2-iC_3H_7$ | $C_{18}H_{31}ClN_2O_6S_2$ | 471,02 | 239 | 91 |
| 1108 | 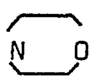 | 2 | $SO_2-iC_3H_7$ | $C_{17}H_{29}ClN_2O_7S_2$ | 472,99 | 198 | 83 |
| 1110 | 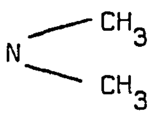 | 3 | $SO_2-iC_3H_7$ | $C_{16}H_{29}ClN_2O_6S_2$ | 444,98 | 162 | 68 |
| 1111 | 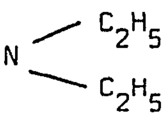 | 3 | $SO_2-iC_3H_7$ | $C_{18}H_{33}ClN_2O_6S_2$ | 473,08 | 172 | 94 |
| 1112 | 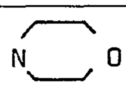 | 3 | $SO_2-iC_3H_7$ | $C_{18}H_{31}ClN_2O_7S_2$ | 487,01 | 185 | 68 |
| 1113 | 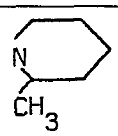 | 3 | $SO_2-iC_3H_7$ | $C_{20}H_{35}ClN_2O_6S_2$ | 499,07 | 200 | 65 |

Les nouveaux benzènesulfonamides selon l'invention, ainsi que leurs sels organiques ou minéraux physiologiquement acceptables, présentent de remarquables propriétés pharmacologiques.

Les composés selon l'invention exercent une action régulatrice sur le système nerveux central et notamment une action psychomodulatrice. Ils peuvent agir, notamment, soit comme antidépresseurs, soit comme anxiolytiques tranquillisants. L'activité de ces composés est renforcée par le caractère lipophile de la molécule, dû à la présence d'un groupe alcoxy sur le noyau aromatique en position 4.

Les composés selon l'invention se distinguent aussi par le fait qu'ils potentialisent le sommeil au pentobarbital, qu'ils ne présentent pas ou peu d'affinité pour les sites dopaminergiques habituellement reconnus comme liés à certains effets indésirables (galactorrhées, aménorrhées, syndromes extrapyramidaux), et qu'ils sont dépourvus de toxicité.

Leur indice thérapeutique est compatible avec leur utilisation comme médicament.

Les composés sont avantageusement introduits comme principe actif dans le traitement des maladies à composantes dépressive, anxieuse et provoquant notamment des troubles psychosomatiques.

Les composés selon l'invention sont à cet effet conditionnés avec les excipients et adjuvants traditionnels, notamment ceux utilisés pour la prépartion de comprimés, poudres, gélules, ampoules buvales, solutions injectables.

L'administration des médicaments contenant les composés selon l'invention est effectuée de

préférence par voie orale, parentérale, rectale ou locale, et les doses de composé actif adminsitrées sont de préférence comprises entre 10 et 700 et notamment entre 50 et 500 mg/d.

A titre d'exemple, différents essais pour la mise en évidence des propriétés pharmacologiques des composés selon l'invention sont rapportés ci-après.

*Essais relatifs à l'étude des interactions des médicaments selon l'invention avec le pentobarbital*

Les essais sont réalisés sur des souris Swiss, EOPS de sexe mâle, pesant de 20 à 24 g, en provenance du centre d'élevage de Le Genest.

Les animaux sont acclimatés au moins 8 d à l'animalerie du laboratoire avant les essais.

Les lots sont de 10 souris par test et par produit.

A titre de produits de référence, pour effectuer les essais témoins, on a utilisé les trois substances suivantes:

N-[(éthyl-1-pyrrolidinyl-2)-méthyl]méthoxy-2-sulfamoyl-5-benzamide, connue sous la désignation sulpiride;

N-[(éthyl-1-pyrrolidinyl-2)-méthyl]méthoxy-2-éthylsulfonyl-5-benzamide, connue sous la désignation sultopride, et

N-(diéthylamino-2-éthyl)méthoxy-2-méthylsulfonyl-5-benzamide, connue sous la désignation tiapride.

Les composés selon l'invention et les substances de référence sont administrés en suspension aqueuse dans l'eau ordinaire à 3% en gomme arabique pour la voie orale (tests d'interactions avec les barbituriques).

Le réactif pharmacologique, à savoir le pentobarbital, est administré en solution dans du soluté isotonique de NaCl.

Les volumes administrés sont de 0,5ml d'une solution à 1,6 g/l pour 20 g de poids corporel, par voie intrapéritonéale (40 mg/kg).

Pour effectuer ces essais, on administre à des lots de 10 souris Swiss mâles les composés selon l'invention ou les produits de référence (à savoir sulpiride, sultopride ou tiapride) par voie orale, à raison de 200 mg/kg. 60 min après l'ingestion des composés selon l'invention ou des produits de référence, on administre aux animaux le pentobarbital sous forme sodique, tel que celui commercialisé sous la marque Nembutal. On mesure ensuite:

– le temps d'endormissement,

– le temps de sommeil,

et on détermine le pourcentage de variation en plus ou en moins du temps de sommeil. Les résultats de ces essais figurent dans le tableau II.

*Tableau II*

| Produit N° | Temps de sommeil (pentobarbital) (min) | Variation (%) |
|---|---|---|
| 880 | | + 55,6 |
| 881 | | + 30,8 |
| 882 | 176±21 | +144,4 |
| 883 | 176±12 | +147,9 |
| 884 | 150±24 | +111,3 |
| 886 | | + 36,4 |
| 887 | | + 24,7 |
| 888 | 140±20 | + 97,2 |
| 889 | 156±15 | +119,7 |
| 897 | | – 8,8 |
| 898 | | – 15,4 |
| 899 | | + 23,1 |
| 900 | | + 14,3 |
| 901 | | + 11,0 |
| 893 | | + 3,3 |
| 891 | | – 7,7 |
| 894 | | + 12,1 |
| 895 | | + 67,1 |
| 924 | | + 16,5 |
| 925 | | + 29,4 |
| 926 | 168±30 | +133,3 |
| 927 | 169±26 | +134,7 |
| 928 | 165±24 | +129,2 |
| 930 | | + 52,8 |
| 931 | | + 28,6 |
| 932 | 142±15 | + 97,2 |
| 933 | 180±32 | +150,0 |
| 955 | | + 11,7 |
| 956 | | – 26,4 |
| 957 | | + 26,8 |
| 958 | | + 16,9 |
| 959 | | + 41,7 |
| 961 | | + 15,6 |
| 962 | | + 11,0 |
| 963 | | – 5,2 |
| 964 | | + 16,9 |
| 979 | | – 8,5 |
| 980 | | + 21,1 |
| 981 | | + 9,1 |
| 982 | | + 2,6 |
| 983 | | – 5,2 |
| 985 | | + 20,8 |
| 986 | | + 16,9 |
| 987 | | + 9,9 |
| 988 | | + 9,9 |

Les nouveaux benzènesulfonamides trisubstitués conformes à l'invention constituent une classe de produits et une classe de médicaments particulièrement préférées.

Les benzènesulfonamides disubstitués présentent des propriétés intéressantes mais, compte tenu des résultats obtenus, il résulte que, lorsque $R_5$ est différent de l'hydrogène, les propriétés biologiques des benzènesulfonamides trisubstitués sont amplifiées par rapport à celles des benzènesulfonamides disubstitués correspondants.

Par conséquent, la combinaison de la disubstitution en (2,4)- par des groupes alcoxy et de la substitution en position 5 est particulièrement

favorable vis-à-vis de l'activité globale de ces molécules.

L'étude des résultats obtenus montre que les composés selon l'invention sont des psychomodulateurs. Ceux pour lesquels le pourcentage de la variation du temps de sommeil est supérieur à +50% sont des potentialisateurs très nets du sommeil au pentobarbital et, par conséquent, peuvent être utilisés comme tranquillisants ou anxiolytiques.

Les composés selon l'invention pour lesquels le pourcentage de la variation du temps de sommeil varie d'environ 0 à 50% sont des potentialisateurs d'activité voisins des composés de référence utilisés sultopride et tiapride.

Les composés selon l'invention pour lesquels le pourcentage de la variation du temps de sommeil est inférieur à 0% sont des antagonistes et peuvent être utilisés comme antidépresseurs.

On note que le sulpiride utilisé à titre de produit de référence se révèle être tantôt potentialisateur, tantôt antagoniste, ce qui introduit son double profil de neuroleptique et d'antidépresseur.

**Revendications** pour les Etats contractants BE, CH, DE, GB, IT, LI, LU, NL, SE

1. Composés répondant à la formule générale (I):

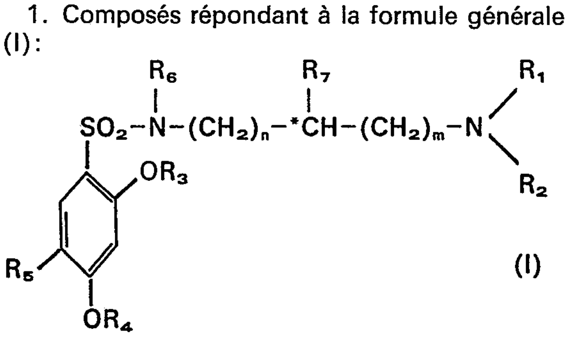

(I)

dans laquelle:

n et m, indépendamment l'un de l'autre, prennent les valeurs de 0 à 4;

$R_3$ et $R_4$ représentent chacun, indépendamment l'un de l'autre, un radical alcoyle inférieur présentant de 1 à 4 atomes de carbone;

$R_1$ et $R_2$ représentent, indépendamemnt l'un de l'autre, des atomes d'hydrogène, des groupes alcoyle linéaires ou ramifiés ayant de 1 à 4 atomes de carbone, ou formant conjointement avec l'atome d'azote un hétérocycle azoté à 5 ou 6 chaînons, notamment un groupe pyrrolidino, morpholino, pipérazinyle, pyrrole ou pipéridino, substitués ou non par des radicaux alcoyle linéaires ou ramifiés ayant de 1 à 4 atomes de carbone;

$R_5$ représente un atome d'hydrogène, un halogène, le groupe $NO_2$, $NH_2$ ou $CF_3$, un radical alcoxy ayant de 1 à 4 atomes de carbone, ou un radical alcoylsulfonyle ayant de 1 à 4 atomes de carbone, et

$R_6$ et $R_7$ représentent, indépendamment l'un de l'autre un atome d'hydrogène, un radical alcoyle ayant de 1 à 6 atomes de carbone ou un radical cycloalcoyle ayant de 3 à 6 atomes de carbone,

ainsi que leurs sels obtenus avec des acides organiques ou minéraux physiologiquement acceptables.

2. Composés selon la revendication 1 répondant à la formule générale (II):

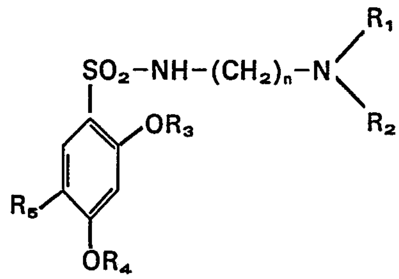

(II)

dans laquelle:

n vaut 2 ou 3;

$R_3$ et $R_4$ représentent chacun, indépendamment l'un de l'autre, un radical alcoyle inférieur présentant de 1 à 4 atomes de carbone;

$R_1$ et $R_2$ représentent, indépendamment l'un de l'autre, des atomes d'hydrogène, des groupes alcoyle linéaires ou ramifiés ayant de 1 à 4 atomes de carbone, ou formant conjointement avec l'atome d'azote un hétérocycle azoté à 5 ou 6 chaînons, notamment un groupe pyrrolidino, morpholino, pipérazinyle, pyrrole ou pipéridino, substitués ou non par des radicaux alcoyle linéaires ou ramifiés ayant de 1 à 4 atomes de carbone, et

$R_5$ représente un atome d'hydrogène, un halogène, le groupe $NO_2$, $NH_2$ ou $CF_3$, un radical alcoxy ayant de 1 à 4 atomes de carbone, ou un radical alcoylsulfonyle ayant de 1 à 4 atomes de carbone,

ainsi que leurs sels obtenus avec des acides organiques ou minéraux physiologiquement acceptables.

3. Composés selon la revendication 2, de formule générale (III):

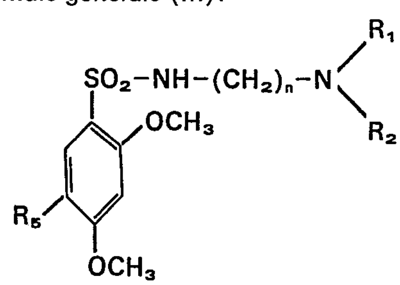

(III)

dans laquelle:

n vaut 2 ou 3;

$R_5$ représente le groupe $OCH_3$, Cl, Br, $SO_2CH_3$, $SO_2C_2H_5$, $SO_2nC_3H_7$ ou $SO_2iC_3H_7$;

$N(R_1)(R_2)$ représente l'un des radicaux choisis dans le groupe comprenant les radicaux diméthylamino, diéthylamino, pyrrolidino, morpholino, pipéridino et pipéridino substitué par un radical méthyle, notamment en position 2,

ainsi que leur sels organiques ou minéraux physiologiquement acceptables.

4. Composés selon la revendication 3, dans lesquels $R_5$ représente l'hydrogène.

5. Composés selon la revendication 4, dans lesquels:

n vaut 2 ou 3;

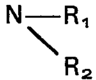 représente l'un des radicaux choisis dans le groupe comprenant les radicaux diméthylamino, diéthylamino, pyrrolidino, pipéridino, morpholino et pipéridino substitué par un groupe méthyle, notamment en position 2.

6. Composés selon la revendication 3, dans lesquels:

$R_5$ représente un halogène, le groupe $NO_2$, $NH_2$ ou $CF_3$, un radical alcoxy ayant de 1 à 4 atomes de carbone, ou un radical alcoylsulfonyle ayant de 1 à 4 atomes de carbone.

7. Composés selon la revendication 6, dans lesquels:

n vaut 2 ou 3;

$R_5$ représente le groupe $OCH_3$, Cl, Br, $SO_2CH_3$, $SO_2C_2H_5$, $SO_2nC_3H_7$ ou $SO_2iC_3H_7$, et

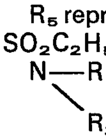 représente l'un des radicaux choisis dans le groupe comprenant les radicaux diméthylamino, diéthylamino, pyrrolidino, morpholino, pipéridino et pipéridino substitué par un radical méthyle, notamment en position 2,

ainsi que leurs sels organiques ou minéraux physiologiquement acceptables.

8. Procédé de préparation du chlorhydrate des composés selon les revendications 1 à 7, caractérisé en ce qu'on fait réagir une amine de formule (XI):

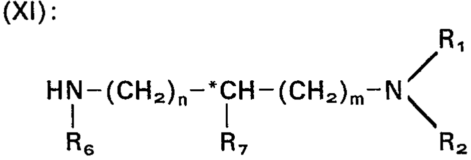

(XI)

dans laquelle:

n et m, indépendamment l'un de l'autre, prennent les valeurs de 0 à 4;

$R_1$ et $R_2$ sont des atomes d'hydrogène, des groupes alcoyle linéaires ou ramifiés ayant de 1 à 4 atomes de carbone, ou formant conjointement avec l'atome d'azote un hétérocycle azoté à 5 ou 6 chaînons, notamment un groupe pyrrolidino, morpholino, pipérazinyle, pyrrole ou pipéridino, substitués ou non par des radicaux alcoyle linéaires ou ramifiés ayant de 1 à 4 atomes de carbone, et

$R_6$ et $R_7$ représentent, indépendamment l'un de l'autre, un atome d'hydrogène, un radical alcoyle ayant de 1 à 6 atomes de carbone ou un radical cycloalcoyle ayant de 3 à 6 atomes de carbone,

sur un sulfochlorure de formule (V):

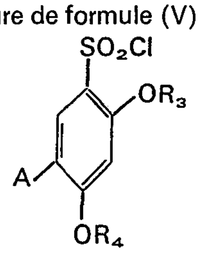

(V)

dans laquelle:

$R_3$ et $R_4$ représentent chacun, indépendamment l'un de l'autre, un radical alcoyle inférieur ayant de 1 à 4 atomes de carbone, et

A a la même signification que $R_5$ selon la revendication 1, à l'exception de $NH_2$,

réaction suivie éventuellement d'une réduction lorsque A représente le groupe $NO_2$.

9. Procédé de prépartion des composés ou de leurs sels organiques ou minéraux physiologiquement acceptables, selon l'une des revendications 1 à 7, caractérisé en ce que le produit obtenu sous forme de chlorhydrate selon la revendication 8 est transformé en la base correspondante ou bien transformé en un sel différent, suivi éventuellement d'une réduction lorsque A représente le groupe $NO_2$.

10. Procédé de préparation selon l'une des revendications 8 ou 9, caractérisé en ce que l'amine est choisie parmi:

la diméthylaminoéthylamine,
la diéthylaminoéthylamine,
la pyrrolidinoéthylamine,
la pipéridinoéthylamine,
la morpholinoéthylamine,
la diméthylaminopropylamine,
la diéthylaminopropylamine,
la pipéridinopropylamine,
la (méthyl-2-pipéridino)-3-propylamine,
la morpholinopropylamine.

11. Procédé de préparation selon l'une des revendications 8 à 10, caractérisé en ce que le sulfochlorure de formule (V) est obtenu à partir des arylamines suivantes:

diméthoxy-2,4-aniline,
chloro-5-diméthoxy-2,4-aniline,
bromo-5-diméthoxy-2,4-aniline,
triméthoxy-2,4,5-aniline,
diméthoxy-2,4-méthylsulfonyl-5-aniline,
diméthoxy-2,4-éthylsulfonyl-5-aniline,
diméthoxy-2,4-propylsulfonyl-5-aniline,
diméthoxy-2,4-isopropylsulfonyl-5-aniline.

12. Composition pharmaceutique, caractérisée en ce qu'elle contient l'un des composés ou leurs sels organiques ou minéraux physiologiquement acceptables selon l'une des revendications 1 à 7.

**Revendications** pour l'Etat contractant: AT

1. Procédé pour préparer les chlorhydrates des composés de formule générale (I):

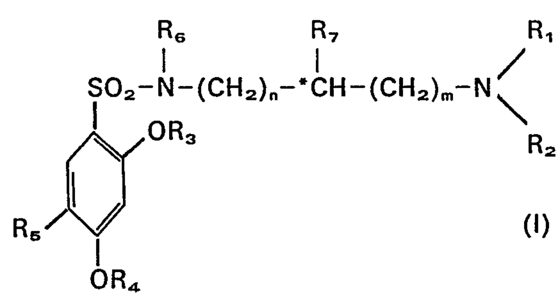

(I)

dans laquelle:

n et m, indépendamment l'un de l'autre, prennent les valeurs de 0 à 4;

$R_3$ et $R_4$ représentent chacun, indépendamment l'un de l'autre, un radical alcoyle inférieur présentant de 1 à 4 atomes de carbone;

$R_1$ et $R_2$ représentent, indépendamment l'un de l'autre, des atomes d'hydrogène, des groupes alcoyle linéaires ou ramifiés ayant de 1 à 4 atomes de carbone, ou formant conjointement avec l'atome d'azote un groupe hétérocycle azoté à 5 ou 6 chaînons, particulièrement un groupe pyrrolidino, morpholino, pipérazinyle, pyrrole ou pipéridino, substitués ou non par des radicaux alcoyle linéaires ou ramifiés ayant de 1 à 4 atomes de carbone;

$R_5$ représente un atome d'hydrogène, un halogène, le groupe $NO_2$, $NH_2$ ou $CF_3$, un radical alcoxy ayant de 1 à 4 atomes de carbone, ou un radical alcoylsulfonyle ayant de 1 à 4 atomes de carbone, et

$R_6$ et $R_7$ représentent, indépendamment l'un de l'autre, un atome d'hydrogène, un radical alcoyle ayant de 1 à 6 atomes de carbone ou un radical cycloalcoyle ayant de 3 à 6 atomes de carbone,

ainsi que leurs sels obtenus avec des acides organiques ou minéraux physiologiquement acceptables, comprenant la réaction d'une amine de formule (XI):

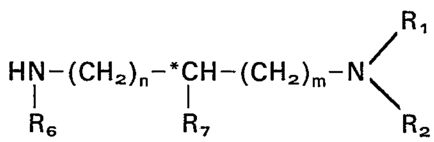

(XI)

dans laquelle:

n et m, indépendamment l'un de l'autre, prennent les valeurs de 0 à 4;

$R_1$ et $R_2$ représentent des atomes d'hydrogène, des groupes alcoyle linéaires ou ramifiés ayant de 1 à 4 atomes de carbone, ou formant conjointement avec l'atome d'azote un groupe hétérocycle azoté à 5 ou 6 chaînons, particulièrement un groupe pyrrolidino, morpholino, pipérazinyle, pyrrole ou pipéridino, morpholino, pipérazinyle, pyrrole ou pipéridino substitués ou non par des radicaux alcoyle linéaires ou ramifiés ayant de 1 à 4 atomes de carbone, et

$R_6$ et $R_7$ représentent, indépendamment l'un de l'autre, un atome d'hydrogène, un radical alcoyle ayant de 1 à 6 atomes de carbone ou un radical cycloalcoyle ayant de 3 à 6 atomes de carbone,

avec un sulfochlorure de formule (V):

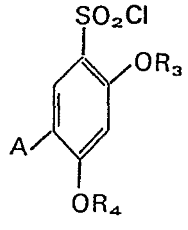

(V)

dans laquelle:

$R_3$ et $R_4$ représentent chacun, indépendamment l'un de l'autre, un radical alcoyle inférieur de 1 à 4 atomes de carbone, et

A a la même signification que $R_5$, à l'exception de $NH_2$,

réaction suivie, si nécessaire, d'une réduction lorsque A représente le groupe $NO_2$.

2. Procédé selon la revendication 1, pour préparer les chlorhydrates des composés de formule générale (II):

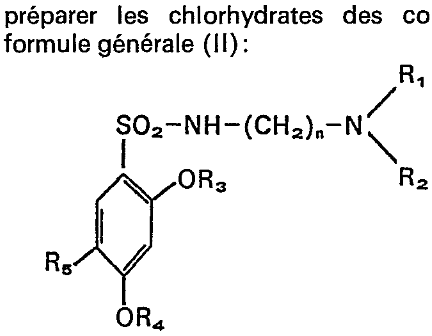

(II)

dans laquelle:

n vaut 2 ou 3;

$R_3$ et $R_4$ représentent chacun, indépendamment l'un de l'autre, un radical alcoyle inférieur ayant de 1 à 4 atomes de carbone;

$R_1$ et $R_2$ représentent, indépendamment l'un de l'autre, des atomes d'hydrogène, des groupes alcoyle linéaires ou ramifiés ayant de 1 à 4 atomes de carbone, ou formant conjointement avec l'atome d'azote un groupe hétérocycle azoté à 5 ou 6 chaînons, particulièrement un groupe pyrrolidino, morpholino, pipérazinyle, pyrrole ou pipéridino, substitués ou non par des radicaux alcoyle linéaires ou ramifiés ayant de 1 à 4 atomes de carbone, et

$R_5$ représente un atome d'hydrogène, un halogène, $NO_2$, $NH_2$, un groupe $CF_3$, un radical alcoxy ayant de 1 à 4 atomes de carbone, ou un radical alcoylsulfonyle ayant de 1 à 4 atomes de carbone,

ainsi que leurs sels obtenus avec des acides organiques ou minéraux physiologiquement acceptables.

3. Procédé selon la revendication 1, pour préparer les chlorhydrates des composés de formule générale (III):

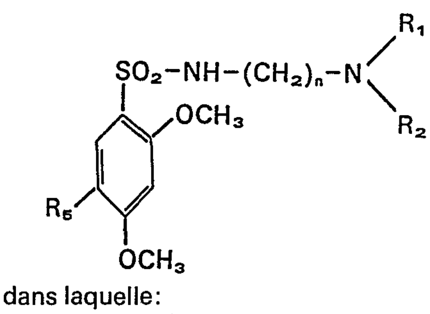

(III)

dans laquelle:

n vaut 2 ou 3;

$R_5$ représente le groupe $OCH_3$, Cl, Br, $SO_2CH_3$, $SO_2C_2H_5$, $SO_2nC_3H_7$ ou $SO_2iC_3H_7$, et

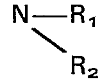 représente l'un des radicaux choisi parmi le groupe comprenant les radicaux diméthylamino, diéthylamino, pyrrolidino, morpholino, pipéridino et pipéridino substitué par un radical méthyle, notamment en position 2,

ainsi que leurs sels organiques ou minéraux physiologiquement acceptables.

4. Procédé selon la revendication 1, pour préparer les chlorhydrates des composés selon la

revendication 3, dans lesquels $R_5$ représente un hydrogène.

5. Procédé selon la revendication 1, pour préparer les chlorhydrates des composés selon la revendication 4, dans lesquels:

n vaut 2 ou 3;

$R_5$ représente un atome d'hydrogène, et

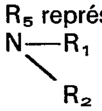 représente l'un des radicaux choisi parmi le groupe comprenant les radicaux diméthylamino, diéthylamino, pyrrolidino, pipéridino, morpholino ou pipéridino substitué par un groupe méthyle, notamment en position 2.

6. Procédé selon la revendication 1, pour préparer les chlorhydrates des composés selon la revendication 3, dans lesquels $R_5$ représente un halogène, un groupe $NO_2$, $NH_2$ ou $CF_3$, un radical alcoxy ayant de 1 à 4 atomes de carbone, ou un radical alcoylsulfonyle ayant de 1 à 4 atomes de carbone.

7. Procédé selon la revendication 1, pour préparer les chlorhydrates des composés selon la revendication 6, dans lesquels:

n vaut 2 ou 3;

$R_5$ représente le groupe $OCH_3$, Cl, Br, $SO_2CH_3$, $SO_2C_2H_5$, $SO_2nC_3H_7$ ou $SO_2iC_3H_7$, et

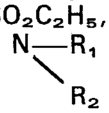 représente l'un des radicaux choisi parmi le groupe comprenant les radicaux diméthylamino, diéthylamino, pyrrolidino, morpholino, pipéridino et pipéridino, substitué par un radical méthyle, notamment en position 2,

ainsi que leurs sels organiques ou minéraux physiologiquement acceptables.

8. Procédé pour préparer les composés, ou leurs sels organiques ou minéraux physiologiquement acceptables, correspondant aux chlorhydrates des composés obtenus selon les revendications 1 à 7, dans lequel ledit chlorhydrate est converti en la base correspondante ou en un sel différent, suivi, si nécessaire, d'une réduction lorsque A représente le groupe $NO_2$.

9. Procédé selon les revendications 1 à 8, dans lequel l'amine est choisie dans le groupe suivant:

diméthylaminoéthylamine,
diéthylaminoéthylamine,
pyrrolidinoéthylamine,
pipéridinoéthylamine,
morpholinoéthylamine,
diméthylaminopropylamine,
diéthylaminopropylamine,
pipéridinopropylamine,
3-(2-méthylpipéridino)propylamine,
morpholinopropylamine.

10. Procédé selon l'une des revendications 1 à 9, dans lequel le sulfochlorure de formule (V) est obtenu à partir des arylamines suivantes:

2,4-diméthoxyaniline,
5-chloro-2,4-diméthoxyaniline,
5-bromo-2,4-diméthoxyaniline,
2,4,5-triméthoxyaniline,
2,4-diméthoxy-5-méthylsulfonylaniline,
2,4-diméthoxy-5-éthylsulfonylaniline,
2,4-diméthoxy-5-propylsulfonylaniline,
2,4-diméthoxy-5-isopropylsulfonylaniline.

11. Procédé de préparation d'une composition pharmaceutique, caractérisé en ce que l'on associe l'un quelconque des composés obtenus à l'issue du procédé défini selon l'une des revendications 1 à 10 avec un véhicule pharmaceutiquement acceptable, dans des conditions permettant la production de ladite composition pharmaceutique.

**Claims** for the Contracting States BE, CH, DE, GB, IT, LI, LU, NL, SE

1. Compounds corresponding to the general formula (I):

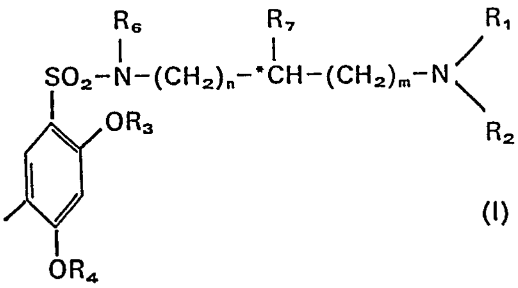

(I)

in which:

n and m, independently from each other, have values from 0 to 4;

$R_3$ and $R_4$ each represent, independently from each other, a lower alkyl radical having from1 to 4 carbon atoms;

$R_1$ and $R_2$ represent, independently from each other, hydrogen atoms, linear or branched alkyl groups having from 1 to 4 carbon atoms, or forming conjointly with the nitrogen atom a nitrogenous heterocyclic group with 5 or 6 links, particularly a pyrrolidino, morpholino, piperazinyl, pyrrole or piperidino group, substituted or not by linear or branched alkyl radicals having from 1 to 4 carbon atoms;

$R_5$ represents a hydrogen atom, a halogen, the group $NO_2$, $NH_2$ or $CF_3$, an alkoxy radical having from1 to 4 carbon atoms, or an alkyl sulfonyl radical having from 1 to 4 carbon atoms, and

$R_6$ and $R_7$ represent, independently from each other, a hydrogen atom, an alkyl radical with 1 to 6 carbon atoms or a cycloalkyl radical with 3 to 6 carbon atoms,

as well as their salts obtained with physiologically acceptable organic or inorganic acids.

2. Compounds according to Claim 1, corresponding to the general formula (II):

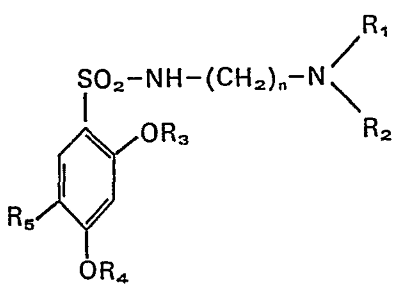

(II)

in which:
n is 2 or 3;
$R_3$ and $R_4$ each represents, independently from each other, a lower alkyl radical having from1 to 4 carbon atoms;
$R_1$ and $R_2$ represent, independently from each other, hydrogen atoms, linear or branched alkyl groups of 1 to 4 carbon atoms, or forming conjointly with the nitrogen atom a nitrogenous heterocyclic group with 5 or 6 links, particularly a pyrrolidino, morpholino, piperazinyl, pyrrole or piperidino group, substituted or not by linear or branched alkyl radicals having from 1 to 4 carbon atoms, and
$R_5$ represents a hydrogen atom, halogen, the $NO_2$, $NH_2$ or $CF_3$ group, an alkoxy radical having from 1 to 4 carbon atoms, or an alkylsuflonyl radical having from 1 to 4 carbon atoms,
as well as their salts obtained with physiologically, acceptable organic or inorganic acids.

3. Compounds according to Claim 2, or the following general formula (III):

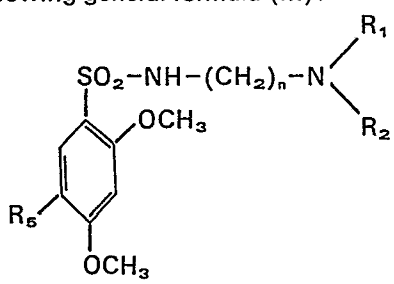

(III)

in which:
n is 2 or 3;
$R_5$ represents the $OCH_3$, Cl, Br, $SO_2CH_3$, $SO_2C_2H_5$, $SO_2nC_3H_7$ or $SO_2iC_3H_7$ group, and
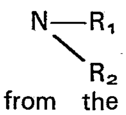 represents one of the radicals selected from the group comprising dimethylamino, diethylamino, pyrrolidino, morpholino, piperidino and piperidino radicals substituted by a methyl radical, particularly at the 2 position,
as well as their physiologically acceptable organic or inorganic salts.

4. Compounds according to Claim 3, in which $R_5$ represents hydrogen.

5. Compounds according to Claim 4, in which:
n is 2 or 3;
$R_5$ represents a hydrogen atom, and
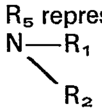 represents one of the radicals selected from the group comprising the dimethylamino, diethylamino, pyrrolidino, piperidino, morpholino and piperidino radicals substituted by a methyl group, particularly at the 2 position.

6. Compounds according to Claim 3, in which $R_5$ represents a halogen, the $NO_2$, $NH_2$ or $CF_3$ group, an alkoxy radical having from 1 to 4 carbon atoms, or an alkylsulfonyl radical having from 1 to 4 carbon atoms.

7. Compounds according to Claim 6 in which:
n is 2 or 3;
$R_5$ represents the $OCH_3$, Cl, Br, $SO_2CH_3$, $SO_2C_2H_5$, $SO_2nC_3H_7$or $SO_2iC_3H_7$ group, and
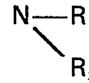 represents one of the radicals selected from the group comprising the dimethylamino, diethylamino, pyrrolidino, morpholino, piperidino and piperidino radicals substituted by a methyl radical, particularly at the 2 position,
as well as their physiologically acceptable organic or inorganic salts.

8. Process for the preparation of the hydrochloride of the compounds according to Claims 1 to 7, comprising reacting an amine of the formula (XI):

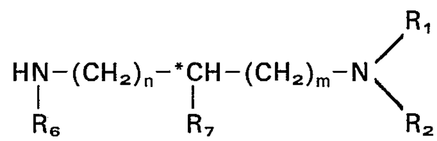

(XI)

in which:
n and m, independently from each other, have values from 1 to 4;
$R_1$ and $R_2$ are hydrogen atoms, linear or branched alkyl groups having from 1 to 4 carbon atoms, or forming conjointly with the nitrogen atom a nitrogenous heterocyclic group with 5 or 6 links, particularly a pyrrolidino, morpholino, piperazinyl, pyrrole or piperidino group, substituted or not by linear or branched alkyl radicals having from 1 to 4 carbon atoms, and
$R_6$ and $R_7$ represent, independently from each other, a hydrogen atom, an alkyl radical having from 1 to 6 carbon atoms or a cycloalkyl radical having from 3 to 6 carbon atoms,
with a sulfochloride of the formula (V):

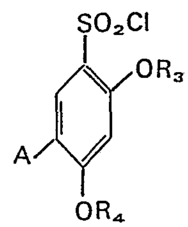

(V)

in which:
$R_3$ and $R_4$ each represents, independently from each other, a lower alkyl radical having from 1 to 4 carbon atoms, and
A has the same meaning as $R_5$ according to Claim 1, with the exception of $NH_2$,
followed, if necessary, by a reduction when A represents the $NO_2$ group.

9. Process for the preparation of the compounds or of their physiologically acceptable organic or inorganic salts, according to any one of Claims 1 to 7, wherein the product obtained in the form of the hydrochloride according to Claim 8 is converted into the corresponding base, or converted into a different salt, followed if necessary by reduction when A represents the $NO_2$ group.

10. Process according to one of Claims 8 or 9, wherein the amine is selected from among:
dimethylamino-ethylamine,
diethylamino-ethylamine,
pyrrolidino-ethylamine,

piperidino-ethylamine,
morpholino-ethylamine,
dimethylamino-propylamine,
diethylamino-propylamine,
piperidino-propylamine,
3-(2-methyl-piperidino)-propylamine,
morpholino-propylamine.

11. Process according to one of Claims 8 to 10, wherein the sulfochloride of formula (V) is obtained from the following arylamines:

2,4-dimethoxy aniline,
5-chloro-2,4-dimethoxy aniline,
5-bromo-2,4-dimethoxy aniline,
2,4,5-trimethoxy aniline,
2,4-dimethoxy-5-methylsulfonyl aniline,
2,4-dimethoxy-5-ethylsulfonyl aniline,
2,4-dimethoxy-5-propylsulfonyl aniline.
2,4-dimethoxy-5-isopropylsulfonyl aniline.

12. Pharmaceutical composition comprising one of the compounds or their physiologically acceptable organic or inorganic salts according to one of Claims 1 to 7.

**Claims** for the Contracting State: AT

1. Process for preparing the hydrochlorides of the compounds of general formula (I):

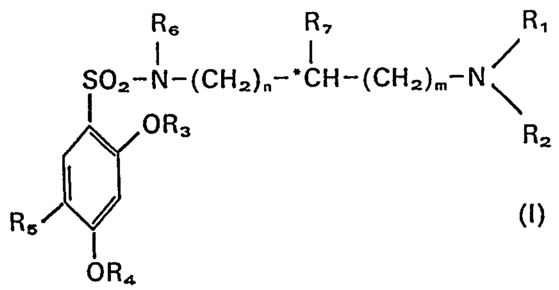
(I)

in which:

n and m, independently from each other, have the values from 0 to 4;

$R_3$ and $R_4$ each represents, independently from each other, an alkyl radical having from 1 to 4 carbon atoms;

$R_1$ and $R_2$ represent, independently from each other, hydrogen atoms, linear or branched alkyl groups having from1 to 4 carbon atoms, or forming conjointly with the nitrogen atom a nirogenous heterocyclic group with 5 or 6 links, particularly a pyrrolidino, morpholino, piperazinyl, pyrrole or piperidino group, substituted or not by linear or branched alkyl radicals having from 1 to 4 carbon atoms;

$R_5$ represents a hydrogen atom, a halogen, the $NO_2$, $NH_2$ or $CF_3$ group, an alkoxy radical having from 1 to 4 carbon atoms or an alkylsulfonyl radical having from 1 to 4 carbon atoms, and

$R_6$ and $R_7$ represent, independently from each other, a hydrogen atom, an alkyl radical having from 1 to 6 carbon atoms or a cycloalkyl radical having from 3 to 6 carbon atoms,

as well as their salts obtained with physiologically acceptable organic or inorganic acids, comprising reacting an amine of formula (XI):

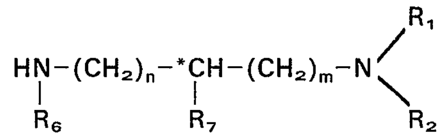
(XI)

in which:

n and m, independently from each other, have values from 1 to 4;

$R_1$ and $R_2$ represent, independently form each other, hydrogen atoms, linear or branched alkyl groups having from 1 to 4 carbon atoms, or forming conjointly with the nitrogen atom a nitrogenous heterocyclic group with 5 or 6 links, particularly a pyrrolidino, morpholino, piperazinyl, pyrrole or piperidino group, substituted or not by linear or branched alkyl radicals having from 1 to 4 carbon atoms, and

$R_6$ and $R_7$ represent, independently from each other, a hydrogen atom, an alkyl radical having from 1 to 6 carbon atoms or a cycloalkyl radical having from 3 to 6 carbon atoms,

with a sulfochloride of formula (V):

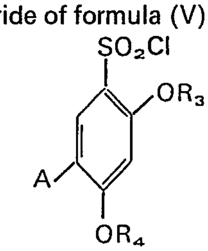
(V)

in which:

$R_3$ and $R_4$ each represents, independently from each other, a lower alkyl radical having from 1 to 4 carbon atoms, and

A has the same meaning as $R_5$, with the exception of $NH_2$,

followed, if necessary, by a reduction when A represents the $NO_2$ group.

2. Process according to Claim 1 for preparing hydrochlorides of compounds of general formula (II):

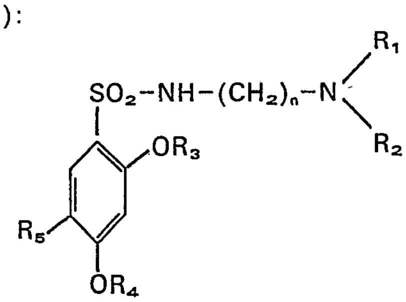
(II)

in which:

n is 2 or 3;

$R_3$ and $R_4$ each represents, independently from each other, a lower alkyl radical having from 1 to 4 carbon atoms, and

$R_1$ and $R_2$ represent, independently from each other, hydrogen atoms, linear or branched alkyl groups of 1 to 4 carbon atoms, or forming conjointly with the nitrogen atom a nitrogenous heterocyclic group with 5 or 6 links, particularly a pyrrolidino, morpholino, piperazinyl, pyrrole or piperidino group, substituted or not by linear or branched alkyl radicals having from 1 to 4 carbon atoms, and

$R_5$ represents a hydrogen atom, a halogen, a $NO_2$, $NH_2$ or $CF_3$ group, an alkoxy radical having from 1 to 4 carbon atoms, or an alkylsulfonyl radical having from 1 to 4 carbon atoms,
as well as their salts obtained with physiologically acceptable organic or inorganic acids.

3. Process according to Claim 1 for preparing hydrochlorides of compounds of general formula (III):

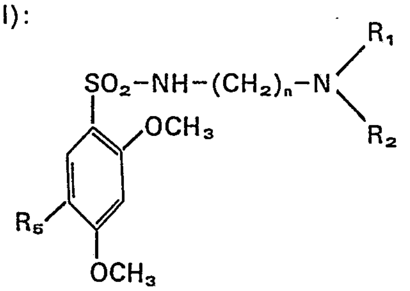

(III)

in which:
n is 2 or 3;
$R_5$ represents the $OCH_3$, Cl, Br, $SO_2CH_3$, $SO_2C_2H_5$, $SO_2nC_3H_7$ or $SO_2iC_3H_7$ group, and
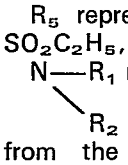 represents one of the radicals selected from the group comprising dimethylamino, diethylamino, pyrrolidino, morpholino, piperidino and piperidino radicals substituted by a methyl radical, particularly at the 2 position,
as well as their physiologically acceptable organic or inorganic salts.

4. Process according to Claim 1 for preparing hydrochlorides of compounds according to Claim 3, in which $R_5$ represents hydrogen.

5. Process according to Claim 1 for preparing hydrochlorides of compounds according to Claim 4, in which:
n is 2 or 3;
$R_5$ represents a hydrogen atom, and
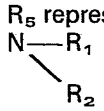 represents one of the radicals selected from the group comprising dimethylamino, diethylamino, pyrrolidino, piperidino, morpholino and piperidino radicals substituted by a methyl group, particularly at the 2 position.

6. Process according to Claim 1 for preparing hydrochlorides of compounds according to Claim 3, in which $R_5$ represents a halogen, the $NO_2$, $NH_2$ or $CF_3$ group, an alkoxy radical having from 1 to 4 carbon atoms, or an alkylsulfonyl radical having from 1 to 4 carbon atoms.

7. Process according to Claim 1 for preparing hydrochlorides of compounds according to Claim 6, in which:
n is 2 or 3;
$R_5$ represents the $OCH_3$, Cl, Br, $SO_2CH_3$, $SO_2C_2H_5$, $SO_2nC_3H_7$ or $SO_2iC_3H_7$ group, and
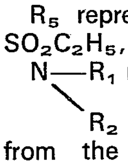 represents one of the radicals selected from the group comprising dimethylamino, diethylamino, pyrrolidino, morpholino, piperidino and piperidino radicals substituted by a methyl radical, particularly at the 2 position,
as well as their physiologically acceptable organic or inorganic salts.

8. Process for preparing compounds or their physiologically acceptable organic or inorganic salts corresponding to hydrochlorides of the compounds obtained according to Claims 1 to 7, in which said hydrochloride is transformed from the corresponding base into a different salt, followed, if necessary, by a reduction when A represents the $NO_2$ group.

9. Process according to Claims 1 to 8, in which the amine is selected from among:
dimethylamino-ethylamine,
diethylamino-ethylamine,
pyrrolidino-ethylamine,
piperidino-ethylamine,
morpholino-ethylamine,
dimethylamino-propylamine,
diethylamino-propylamine,
piperidino-propylamine,
3-(2-methyl-piperidino)-propylamine,
morpholino-propylamine.

10. Process according to Claims 1 to 9, in which the sulfochloride of formula (V) is obtained from the following arylamines:
2,4-dimethoxy aniline,
5-chloro-2,4-dimethoxy aniline,
5-bromo-2,4-dimethoxy aniline,
2,4,5-trimethoxy aniline,
2,4-dimethoxy-5-methylsulfonyl aniline,
2,4-dimethoxy-5-ethylsulfonyl aniline,
2,4-dimethoxy-5-propylsulfonyl aniline,
2,4-dimethoxy-5-isopropylsulfonyl aniline.

11. Process for preparing a pharmaceutical composition, characterized by the fact that any one of the compounds obtained from the process defined according to one of Claims 1 to 10 is associated with a pharmaceutically acceptable vehicle under conditions enabling the production of said pharmaceutical composition.

**Patentansprüche** für die Vertragsstaaten BE, CH, DE, GB,IT, LI, LU, NL, SE

1. Verbindungen der allgemeinen Formel (I):

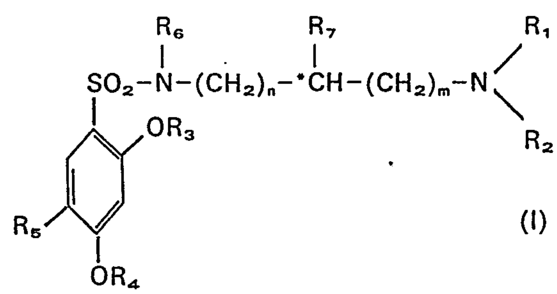

(I)

in welcher:
n und m, unabhängig voneinander, die Werte 0 bis 4 haben;
$R_3$ und $R_4$ einzeln, unabhängig voneinander, einen niedrigen Alkylrest mit 1 bis 4 Kohlenstoffatomen bedeuten;
$R_1$ und $R_2$, unabhängig voneinander, Wasserstoffatome oder gerade oder verzweigte Alkylgruppen mit 1 bis 4 Kohlenstoffatomen sind, oder gemeinsam mit dem Stickstoffatom einen 5- oder 6gliedrigen, stickstoffhaltigen Heterocyclus, insbesondere eine Pyrrolidino-, Morpholino-, Piperazinyl-, Pyrrol- oder Piperidinogruppe, die gegebenenfalls durch gerade oder verzweigte Alkylreste mit 1 bis 4 Kohlenstoffatomen substituiert ist, bilden;

$R_5$ ein Wasserstoffatom, ein Halogen, eine $NO_2$-, $NH_2$- oder $CF_3$-Gruppe, einen Alkoxyrest mit 1 bis 4 Kohlenstoffatomen, oder einen Alkylsulfonylrest mit 1 bis 4 Kohlenstoffatomen bedeutet;

$R_6$ und $R_7$, unabhängig voneinander, ein Wasserstoffatom, einen Alkylrest mit 1 bis 6 Kohlenstoffatomen oder einen Cycloalkylrest mit 3 bis 6 Kohlenstoffatomen bedeuten;

sowie deren physiologisch annehmbare, mit organischen oder anorganischen Säuren erhaltene Salze.

2. Verbindungen nach Anspruch 1, der allgemeinen Formel (II):

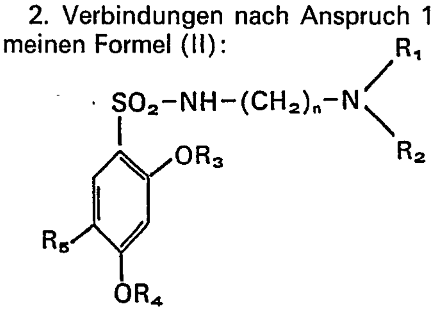

(II)

in welcher:

n 2 oder 3 ist;

$R_3$ und $R_4$ einzeln, unabhängig voneinander, einen niedrigen Alkylrest mit 1 bis 4 Kohlenstoffatomen bedeuten;

$R_1$ und $R_2$, unabhängig voneinander, Wasserstoffatome oder gerade oder verzweigte Alkylgruppen mit 1 bis 4 Kohlenstoffatomen sind, oder gemeinsam mit dem Stickstoffatom einen 5- oder 6gliedrigen, stickstoffhaltigen Heterocyclus, insbesondere eine Pyrrolidino-, Morpholino-, Piperazinyl-, Pyrrol- oder Piperidinogruppe, die gegebenenfalls durch gerade oder verzweigte Alkylreste mit 1 bis 4 Kohlenstoffatomen substituiert ist, bilden;

$R_5$ ein Wasserstoffatom, ein Halogen, eine $NO_2$-, $NH_2$- oder $CF_3$-Gruppe, einen Alkoxyrest mit 1 bis 4 Kohlenstoffatomen, oder einen Alkylsulfonylrest mit 1 bis 4 Kohlenstoffatomen bedeutet;

sowie deren physiologisch annehmbare, mit organischen oder anorganischen Säuren erhaltene Salze.

3. Verbindungen nach Anspruch 2, der folgenden allgemeinen Formel (III):

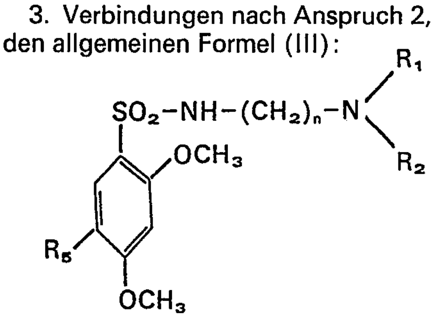

(III)

worin:

n 2 oder 3 ist;

$R_5$ eine $OCH_3$-, Cl-, Br-, $SO_2CH_3$-, $SO_2C_2H_5$-, $SO_2nC_3H_7$- oder $SO_2iC_3H_7$-Gruppe bedeutet;

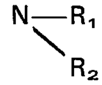 einen aus der die Reste Dimethylamino, Diethylamino, Pyrrolidino, Morpholino, Piperidino, durch einen Methylreste, insbesondere in Stellung 2, substituiertes Piperidino, umfassenden Gruppe ausgewählten Rest bedeutet;

sowie deren physiologisch annehmbare, organische oder anorganische Salze.

4. Verbindungen nach Anspruch 3, in welchen $R_5$ Wasserstoff bedeutet.

5. Verbindungen nach Anspruch 4, in welchen:

n 2 oder 3 ist;

$R_5$ ein Wasserstoffatom bedeutet;

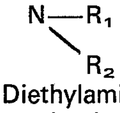 einen aus der die Reste Dimethylamino, Diethylamino, Pyrrolidino, Morpholino, Piperidino, durch einen Methylreste, insbesondere in Stellung 2, substituiertes Piperidino, umfassenden Gruppe ausgewählten Rest bedeutet.

6. Verbindungen nach Anspruch 3, in welchen $R_5$ ein Halogen, eine $NO_2$-, $NH_2$- oder $CF_3$-Gruppe, einen Alkoxyrest mit 1 bis 4 Kohlenstoffatomen, oder einen Alkylsulfonylrest mit 1 bis 4 Kohlenstoffatomen bedeutet.

7. Verbindungen nach Anspruch 6, in welchen:

n 2 oder 3 ist;

$R_5$ eine $OCH_3$-, Cl-, Br-, $SO_2CH_3$-, $SO_2C_2H_5$-, $SO_2nC_3H_7$- oder $SO_2iC_3H_7$-Gruppe bedeutet;

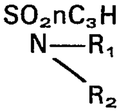 einen aus der die Reste Dimethylamino, Diethylamino, Pyrrolidino, Morpholino, Piperidino, durch einen Methylrest, insbesondere in Stellung 2, substituiertes Piperidino, umfassenden Gruppe ausgewählten Rest bedeutet;

sowie deren physiologisch annehmbare, organische oder anorganische Salze.

8. Verfahren zur Herstellung des Hydrochlorids von Verbindungen der Ansprüche 1 bis 7, dadurch gekennzeichnet, dass man ein Amin der Formel (XI):

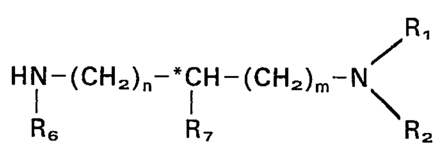

(XI)

in welcher:

n und m, unabhängig voneinander, die Werte 0 bis 4 haben;

$R_1$ und $R_2$ Wasserstoffatome oder gerade oder verzweigte Alkylgruppen mit 1 bis 4 Kohlenstoffatomen sind, oder gemeinsam mit dem Stickstoffatom einen 5- oder 6gliedrigen, stickstoffhaltigen Heterocyclus, insbesondere eine Pyrrolidino-, Morpholino-, Piperazinyl-, Pyrrol- oder Piperidinogruppe, die gegebenenfalls durch gerade oder verzweigte Alkylreste mit 1 bis 4 Kohlenstoffatomen substituiert ist, bilden;

$R_6$ und $R_7$, unabhängig voneinander, ein Wasserstoffatom, einen Alkylrest mit 1 bis 6 Kohlenstoffatomen oder einen Cycloalkylrest mit 3 bis 6 Kohlenstoffatomen bedeuten;
mit einem Sulfonylchlorid der Formel (V):

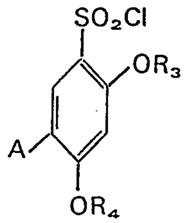

(V)

in welcher:

$R_3$ und $R_4$ einzeln, unabhängig voneinander, einen niedrigen Alkylrest mit 1 bis 4 Kohlenstoffatomen bedeuten;

A die gleiche Bedeutung wie $R_5$ in Anspruch 1, ausgenommen $NH_2$, hat,
umsetzt und gegebenenfalls anschliessend, falls A die Gruppe $NO_2$ bedeutet, eine Reduktion vornimmt.

9. Verfahren zur Herstellung der Verbindungen oder ihrer physiologisch annehmbaren, organischen oder anorganischen Salze, nach einem der Ansprüche 1 bis 7, dadurch gekenzeichnet, dass man das in Form des Hydrochlorids gemäss Anspruch 8 erhaltene Produkt in die entsprechende Base umwandelt, oder aber in ein anderes Salz umwandelt, und, falls A die Gruppe $NO_2$ bedeutet, gegebenenfalls eine Reduktion anschliesst.

10. Verfahren nach einem der Ansprüche 8 oder 9, dadurch gekennzeichnet, dass man als Amin:
Dimethylaminoethylamin
Diethylaminoethylamin
Pyrrolidinoethylamin
Piperidinoethylamin
Morpholinoethylamin
Dimethylaminopropylamin
Diethylaminopropylamin
Piperidinopropylamin
[Methyl-(2)-piperidino]-3-propylamin, oder
Morpholinopropylamin
auswählt.

11. Verfahren nach einem der Ansprüche 8 bis 10, dadurch gekennzeichnet, dass das Sulfonylchlorid der Formel (V) ausgehend von den folgenden Arylaminen erhalten worden ist:
Dimethoxy-(2,4)-anilin
Chlor-(5)-dimethoxy-(2,4)-anilin
Brom-(5)-dimethoxy-(2,4)-anilin
Trimethoxy-(2,4,5)-anilin
Dimethoxy-(2,4)-methylsulfonyl-(5)-anilin
Dimethoxy-(2,4)-ethylsulfonyl-(5)-anilin
Dimethoxy-(2,4)-propylsulfonyl-(5)-anilin, oder
Dimethoxy-(2,4)-isopropylsulfonyl-(5)-anilin.

12. Pharmazeutische Zusammensetzung, dadurch gekennzeichnet, dass sie eine der Verbindungen oder deren physiologisch annehmbare, organische oder anorganische Salze nach einem der Ansprüche 1 bis 7 enthält.

**Patentansprüche** für den Vertragsstaat: AT

1. Verfahren zur Herstellung der Hydrochloride von Verbindungen der allgemeinen Formel (I):

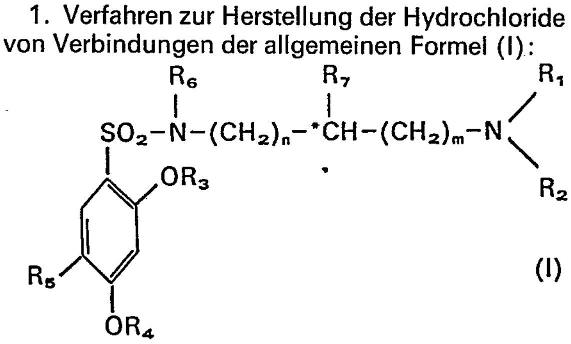

(I)

in welcher:

n und m, unabhängig voneinander, die Werte 0 bis 4 haben;

$R_3$ und $R_4$ einzeln, unabhängig voneinander, einen niedrigen Alkylrest mit 1 bis 4 Kohlenstoffatomen bedeuten;

$R_1$ und $R_2$, unabhängig voneinander, Wasserstoffatome oder gerade oder verzweigte Alkylgruppen mit 1 bis 4 Kohlenstoffatomen sind, oder gemeinsam mit dem Stickstoffatom einen 5- oder 6gliedrigen, stickstoffhaltigen Heterocyclus, insbesondere eine Pyrrolidino-, Morpholino-, Piperazinyl-, Pyrrol- oder Piperidinogruppe, die gegebenenfalls durch gerade oder verzweigte Alkylreste mit 1 bis 4 Kohlenstoffatomen substituiert ist, bilden;

$R_5$ ein Wasserstoffatom, ein Halogen, eine $NO_2$-, $NH_2$- oder $CF_3$-Gruppe, einen Alkoxyrest mit 1 bis 4 Kohlenstoffatomen, oder einen Alkylsulfonylreste mit 1 bis 4 Kohlenstoffatomen bedeutet;

$R_6$ und $R_7$, unabhängig voneinander, ein Wasserstoffatom, einen Alkylrest mit 1 bis 6 Kohlenstoffatomen oder einen Cycloalkylrest mit 3 bis 6 Kohlenstoffatomen bedeuten;
sowie von deren physiologisch annehmbaren, mit organischen oder anorganischen Säuren erhaltenen Salzen, umfassend die Umsetzung eines Amins der Formel (XI):

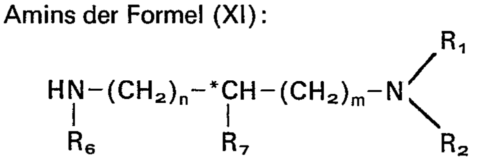

(XI)

in welcher:

n und m, unabhängig voneinander, die Werte 0 bis 4 haben;

$R_1$ und $R_2$ Wasserstoffatome oder gerade oder verzweigte Alkylgruppen mit 1 bis 4 Kohlenstoffatomen sind, oder gemeinsam mit dem Stickstoffatom einen 5- oder 6gliedrigen, stickstoffhaltigen Heterocyclus, insbesondere eine Pyrrolidino-, Morpholino-, Piperazinyl-, Pyrrol- oder Piperidinogruppe, die gegebenenfalls durch gerade oder verzweigte Alkylreste mit 1 bis 4 Kohlenstoffatomen substituiert ist, bilden;

$R_6$ und $R_7$, unabhängig voneinander, ein Wasserstoffatom, einen Alkylrest mit 1 bis 6 Kohlenstoffatomen oder einen Cycloalkylrest mit 3 bis 6 Kohlenstoffatomen bedeuten;
mit einem Sulfonylchlorid der Formel (V):

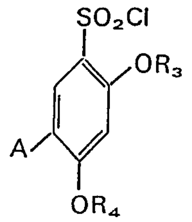

(V)

in welcher:

$R_3$ und $R_4$ einzeln, unabhängig voneinander, einen niedrigen Alkylrest mit 1 bis 4 Kohlenstoffatomen bedeuten;

A die gleiche Bedeutung wie $R_5$, ausgenommen $NH_2$, hat,

und erforderlichenfalls anschliessend, falls A die Gruppe $NO_2$ bedeutet, eine Reduktion.

2. Verfahren nach Anspruch 1 zur Herstellung der Hydrochloride von Verbindungen der allgemeinen Formel (II):

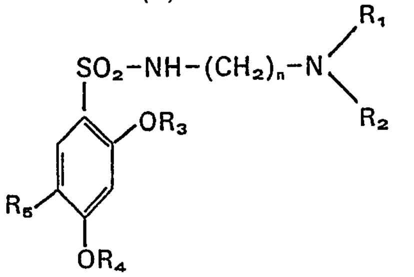

(II)

in welcher:

n 2 oder 3 ist;

$R_3$ und $R_4$ einzeln, unabhängig voneinander, einen niedrigen Alkylrest mit 1 bis 4 Kohlenstoffatomen bedeuten;

$R_1$ und $R_2$, unabhängig voneinander, Wasserstoffatome oder gerade oder verzweigte Alkylgruppen mit 1 bis 4 Kohlenstoffatomen sind, oder gemeinsam mit dem Stickstoffatom einen 5- oder 6gliedrigen, stickstoffhaltigen Heterocyclus, insbesondere eine Pyrrolidino-, Morpholino-, Piperazinyl-, Pyrrol- oder Piperidinogruppe, die gegebenenfalls durch gerade oder verzweigte Alkylreste mit 1 bis 4 Kohlenstoffatomen substituiert ist, bilden;

$R_5$ ein Wasserstoffatom, ein Halogen, eine $NO_2$-, $NH_2$- oder $CF_3$-Gruppe, einen Alkoxyrest mit 1 bis 4 Kohlenstoffatomen, oder einen Alkylsulfonylrest mit 1 bis 4 Kohlenstoffatomen bedeutet;

sowie von deren physiologisch annehmbaren, mit organischen oder anorganischen Säuren erhaltenen Salzen.

3. Verfahren nach Anspruch 1 zur Herstellung der Hydrochloride von Verbindungen der folgenden allgemeinen Formel (III):

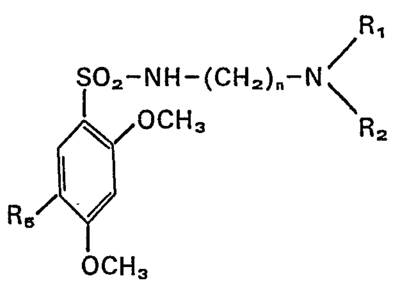

(III)

worin:

n 2 oder 3 ist;

$R_5$ eine $OCH_3$-, Cl-, Br-, $SO_2CH_3$-, $SO_2C_2H_5$-, $SO_2nC_3H_7$- oder $SO_2iC_3H_7$-Gruppe bedeutet;

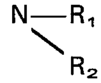 einen aus der die Reste Dimethylamino, Diethylamino, Pyrrolidino, Morpholino, Piperidino, durch einen Methylrest, insbesondere in Stellung 2, substituiertes Piperidino, umfassenden Gruppe ausgewählten Rest bedeutet;

sowie von deren physiologisch annehmbaren, organischen oder anorganischen Salzen.

4. Verfahren nach Anspruch 1 zur Herstellung der Hydrochloride von Verbindungen nach Anspruch 3, in welchen $R_5$ Wasserstoff bedeutet.

5. Verfahren nach Anspruch 1 zur Herstellung der Hydrochloride von Verbindungen nach Anspruch 4, in welchen:

n 2 oder 3 ist;

$R_5$ ein Wasserstoffatom bedeutet;

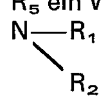 einen aus der die Reste Dimethylamino, Diethylamino, Pyrrolidino, Morpholino, Piperidino, durch einen Methylrest, insbesondere in Stellung 2, substituiertes Piperidino, umfassenden Gruppe ausgewählten Rest bedeutet.

6. Verfahren nach Anspruch 1 zur Herstellung der Hydrochloride von Verbindungen nach Anspruch 3, in welchen $R_5$ ein Halogen, eine $NO_2$-, $NH_2$- oder $CF_3$-Gruppe, einen Alkoxyrest mit 1 bis 4 Kohlenstoffatomen, oder einen Alkylsulfonylrest mit 1 bis 4 Kohlenstoffatomen bedeutet.

7. Verfahren nach Anspruch 1 zur Herstellung der Hydrochloride von Verbindungen nach Anspruch 6, in welchen:

n 2 oder 3 ist;

$R_5$ eine $OCH_3$-, Cl-, Br-, $SO_2CH_3$-, $SO_2C_2H_5$-, $SO_2nC_3H_7$- oder $SO_2iC_3H_7$-Gruppe bedeutet;

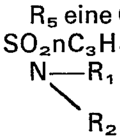 einen aus der die Reste Dimethylamino, Diethylamino, Pyrrolidino, Morpholino, Piperidino, durch einen Methylrest, insbesondere in Stellung 2, substituiertes Piperidino, umfassenden Gruppe ausgewählten Rest bedeutet;

sowie von deren physiologisch annehmbare, organischen oder anorganischen Salzen.

8. Verfahren zur Herstellung der Verbindungen oder ihrer physiologisch annehmbaren, organischen oder anorganischen Salze, die den Hydrochloriden von Verbindungen entsprechen, wie sie nach einem der Ansprüche 1 bis 7 erhalten worden sind, in welchem das genannte Hydrochlorid in die entsprechende Base oder in ein anderes Salz umgewandelt wird, und, falls A die Gruppe $NO_2$ bedeutet, erforderlichenfalls eine Reduktion angeschlossen wird.

9. Verfahren nach den Ansprüchen 1 bis 8, in welchem das Amin aus der folgenden Gruppe:

Dimethylaminoethylamin

Diethylaminoethylamin

Pyrrolidinoethylamin

Piperidinoethylamin

Morpholinoethylamin
Dimethylaminopropylamin
Diethylaminopropylamin
Piperidinopropylamin
3-(2-Methylpiperidino)propylamin, oder
Morpholinopropylamin
ausgewählt wird.

10. Verfahren nach einen der Ansprüche 1 bis 9, in welchem das Sulfonylchlorid der Formel (V) ausgehend von den folgenden Arylaminen erhalten worden ist:
2,4-Dimethoxyanilin
5-Chlor-2,4-dimethoxyanilin
5-Brom-2,4-dimethoxyanilin
2,4,5-Trimethoxyanilin
2,4-Dimethoxy-5-methylsulfonylanilin
2,4-Dimethoxy-5-ethylsulfonylanilin
2,4-Dimethoxy-5-propylsulfonylanilin, oder
2,4-Dimethoxy-5-isopropylsulfonylanilin.

11. Verfahren zur Herstellung von pharmazeutischer Zusammensetzung, dadurch gekennzeichnet, dass irgendeiner der durch das in einem der Patentansprüche 1 bis 10 definierten Verfahren hergestellten Verbindungen ein pharmazeutisch verträgliches Trägermittel zugefügt wird und zwar unter Bedingungen, die die Herstellung der in Frage kommenden pharmazeutischen Zusammensetzung gewährleisten.